# NEUE EUROPÄISCHE PATENTSCHRIFT

(11) **EP 1 575 904 B2**
(45) Veröffentlichungstag und Bekanntmachung des Hinweises auf die Entscheidung über den Einspruch: **04.09.2013**
(45) Hinweis auf die Patenterteilung: 13.10.2010
(21) Anmeldenummer: 03789262.7
(22) Anmeldetag: 13.12.2003
(51) Int. Cl.: C07C 263/10, C07C 265/14

(54) **VERFAHREN ZUR KONTINUIERLICHEN HERSTELLUNG VON ISOCYANATEN**
METHOD FOR THE CONTINUOUS PRODUCTION OF ISOCYANATES
PROCEDE DE FABRICATION EN CONTINU D'ISOCYANATES

(30) Priorität: 19.12.2002 DE 10260082
(43) Veröffentlichungstag der Anmeldung: 21.09.2005
(73) Patentinhaber: BASF SE, 67056 Ludwigshafen (DE)
(72) Erfinder: SOHN, Martin, 68229 Mannheim (DE); STROEFER, Eckhard, 68163 Mannheim (DE); NEVEJANS, Filip, B-9170 St.Gillis-Waas (BE); PENZEL, Ulrich, 01945 Tettau (DE); PALLASCH, Hans-Jürgen, 67169 Kallstadt (DE); LEUTHOLD, Rene, 01945 Hohenbocka (DE); BRODHAGEN, Andreas, 67125 Dannstadt-Schauernheim (DE); WÖLFERT, Andreas, 74906 Bad Rappenau (DE); MACKENROTH, Wolfgang, 67089 Bad Dürkheim (DE); MAURER, Markus, 67071 Ludwigshafen (DE)
(86) Internationale Anmeldenummer: PCT/EP2003/014185
(87) Internationale Veröffentlichungsnummer: WO 2004/056756

(56) Entgegenhaltungen:
- EP-A- 0 065 727
- EP-A- 0 716 079

## Beschreibung

Die Erfindung betrifft ein Verfahren zur Herstellung von aromatischen oder aliphatischen Isocyanaten. Bei den aromatischen Isocyanaten sind dies bevorzugt Methylendi(phenylisocyanat) (MDI) und Toluylendiisocyanat (TDI), bei den aliphatischen Hexamethylendiisocyanat (HDI) und Isophorondiisocyanat (IPDI) oder andere.

Ziel der vorliegenden Erfindung ist es, ein drei- oder mehrstufiges Verfahren zu entwickeln, welches Isocyanate mit sehr hohen chemischen Ausbeuten und hohen Raum-Zeit-Ausbeuten bei niedrigem Phosgen-Holdup liefert.

Beim erfindungsgemäßen Verfahren wird die Reaktion zwischen organischem Amin und Phosgen drei- oder mehrstufig, in einem inerten Lösungsmittel, vorzugsweise Toluol, Chlorbenzol, Dichlorbenzol oder Mischungen der beiden letzteren, und mit einem Phosgenüberschuß durchgeführt, **dadurch gekennzeichnet, dass** über jede der Stufen eine Reduzierung des Drucks erfolgt und die erste Phospenierstufe eine Düse umfaßt die zweite Stufe einen Verweilzeitapparat und die dritte Stufe in einer oder mehreren Reaktions kolonnen durch geführt wird. Der Druck vor der Düse beträgt 3-70 bar, bevorzugt 15-45 bar. Die Verweilzeitraktor der zweiten Stufe wird bei einem Druck von 2,5-35 bar, bevorzugt 15-35 bar betrieben. Hinter der Düse wird über eine Armatur oder eine andere dafür geeignete Einrichtung auf den Druck der zweiten Stufe entspannt. Es kann allerdings auch der natürliche Druckverlust der Düse zur Druckreduzierung verwendet werden. Der Reaktor der ersten Stufe, kann auch in den Reaktor der zweiten Stufe, einen Verweilzeitapparat, integriert werden. Der Reaktor der dritten Phosgenierstufe, eine Reaktionskolonne, wird bei einem Druck von 2-20 bar, vorzugsweise 3,5-16 bar, betrieben. Hinter dem Reaktor der zweiten Stufe wird über eine Armatur oder eine andere dafür geeignete Einrichtung auf den Druck des Reaktors der dritten Stufe entspannt. Gegebenenfalls ist auch ein natürlicher Druckverlust zur Druckreduzierung ausreichend. Als Reaktor der dritten Stufe wird eine Reaktions kolonne verwendet, wie sie beispielsweise in WO 99/54289 (DE 19817691) beschrieben ist.

Es ist bekannt, Isocyanate aus Aminen und Phosgen herzustellen. Die Umsetzung wird je nach Art der Amine entweder in der Gasphase oder in flüssiger Phase sowohl diskontinuierlich als auch kontinuierlich durchgeführt (W. Siefken. Liebigs Ann. 562, 75 (1949)). Die kontinuierliche Herstellung von organischen Isocyanaten durch Reaktion von primären organischen Aminen mit Phosgen ist vielfach beschrieben und wird im großtechnischen Maßstab durchgeführt (s. z.B. Ullmanns Enzyklopädie der Technischen Chemie, Band 7 (Polyurethane), 3. neubearbeitete Auflage, Carl Hanser Verlag, München-Wien, S. 76ff (1993).). Insbesondere die aromatischen Isocyanate TDI (Toluylendiisocyanat) und MDI (Methylendiphenyldiisocyanat) bzw. PMDI (Polymethylenpolyphenylenpolyisocyanat) und Mischungen der letzteren beiden sowie die aliphatischen Isoyanate HDI (Hexamethylendiisocyanat) und Isophorondiisocyanat (IPDI) werden großtechnisch global verteilt hergestellt.

Heutige großtechnische Synthesen von den aromatischen Diisocyanaten MDI und TDI und den aliphatischen Diisocyanaten HDI und IPDI erfolgen fast ausschließlich in kontinuierlichen Verfahren. Ein kontinuierliches Verfahren zur Durchführung der Reaktion in mehreren, kontinuierlich durchströmten Gefäßen findet sich z.B. in DE 844896. In der Regel ist die kontinuierliche Ausführungsform des Verfahrens zweistufig. In der ersten Stufe der Phosgenierung wird das Amin mit Phosgen zum entsprechenden Carbamoylchlorid und Chlorwasserstoff und zu Aminhydrochlorid umgesetzt. Die Reaktion zwischen Amin und Phosgen ist sehr schnell, stark exotherm und läuft schon bei sehr niedrigen Temperaturen ab. Um Nebenprodukte- und Feststoffbildung zu minimieren, müssen daher Amin und Phosgen, beide gegebenenfalls mit organischem Lösungsmittel, schnell vermischt werden, weswegen die erste Phosgenierstufe in der Regel in einem Mischorgan, vorzugsweise einer Düse, erfolgt. Die zweite Stufe der Phosgenierung umfaßt sowohl die Zersetzung des Carbamoylchlorids zum gewünschten Isocyanat und Chlorwasserstoff als auch die Phosgenierung des Aminhydrochlorids zum Carbamoylchlorid. Die Temperatur der zweiten Phosgenierstufe ist in der Regel höheren als die der ersten.

Die Reaktion von Amin und Phosgen in der Flüssigphase ist bei allen technisch üblichen Temperaturen und Drücken sehr schnell. Daher wird eine gute Vermischung der Reaktanden angestrebt, um Nebenreaktionen zu unterdrücken. Die Phosgenierung primärer Amine in einem Mischreaktor als erster Stufe der Phosgenierung ist vielfach beschrieben worden.

Mischorgane können grundsätzlich unterschieden werden in dynamische Mischer wie z.B. Rührer und Turbinen und statische Mischer, Rotor-Stator-Systeme wie z.B. Kenics-Mischer, Schaschlik-Mischer, SMV-Mischer und Strahlmischer wie z.B. Düsen oder T-Mischer (Chem. Ing. Tech. MS 1708/88, Fortschr. Verf. Technik 23, 1985, 373, Ind. Eng. Chem. Res. 26, 1987, 1184).

Zu den bekannten Mischaggregaten gehören vor allem Düsen wie beispielsweise Ringschlitzdüse (DE 1792660), Ringlochdüsen (DE 3744001), Glattstrahlmischdüsen (EP 0065727), Fächerstrahldüsen (DE 2950216), Winkelstrahlkammerdüsen (DD 300.168), Dreistromdüsen (DD 132340), Gegenstrommischkammern (DE-PS 1146872), Staudüsen (FR 69428), Venturimischdüsen (DE-AS 1175666). Auch Inlinemischer (US 3321283), Kreisel- oder Reaktionsmischpumpen (EP 0291819), Tubularreaktoren (US 3226410) oder Mikrostrukturmischer (EP 0928785) sind bekannt. In CA 832432 wird die Verwendung von Schallwellen zur Durchmischung beschrieben.

In EP 0830894 wird ein Mischerreaktor zur Phosgenierung von primären Aminen beschrieben, bei dem der Einlass für den einen Stoff in der Achse der Mischkammer und der Einlass des (mindestens einen) anderen Stoffes in Form einer Vielzahl von rotationssymmetrischen zur Achse der Mischkammer angeordneten Düsen ausgebildet ist, wobei jede dieser Düsen einen in Richtung der Düsenachse verschiebbaren Bolzen besitzt, der die Düse von anhaftenden Feststoffen befreien kann.

In DD 132340 wird ein Verfahren zur Phosgenierung von Aminen zu Mono-, Di- und Polyisocyanaten unter Druck und erhöhter Temperatur in Anwesenheit eines einheitlichen Lösungsmittels beschrieben, wobei ein Amin-Monochlorbenzolgemisch und ein Phosgen-Monochlorbenzolgemisch in mehrere Teilströme aufgeteilt parallel einem Reaktor zugeführt werden, wobei ein Teil des Phosgen-Monochlorbenzolgemisches zentral und um diesen zentralen Strom des Amin-Monochlorbenzolgemisch geführt wird, das wiederum von einem Phosgen-Monochlorbenzolgemisch eingeschlossen wird. Das Polyamin-Monochlorbenzolgemisch wird beispielsweise bei 150°C ringförmig in den Phosgenierreaktor eingespeist. Vor Eintritt in den Reaktor wird das Gemisch durch eine entsprechende Dralleinrichtung in eine rotierende Bewegung versetzt. In und um das Polyamin-Monochlorbenzolgemisch wird als Reaktionspartner ein auf 150°C aufgeheiztes Phosgen-Monochlorbenzolgemisch in den Reaktor geführt. Die Relativgeschwindigkeit zwischen beiden Reaktionspartnern beträgt ca. 15 m/s.

Auch für die zweite Phosgenierstufe, die gegebenenfalls gleichzeitig als Phasentrennbehälter verwendet werden, hat sich eine Vielzahl von Apparaten etabliert. Die Herstellung von Isocyanaten aus den entsprechenden Aminen durch Phosgenierung erfolgt in Rührkesseln (z.B. DE-OS 1468445), in Rührkesselkaskaden (DE-PS 844896), in mit Füllkörpern gefüllten Reaktionssäulen oder Reaktionskolonnen (z.B. WO 99/54289) oder in ungefüllten Kolonnen (z.B. Ullmanns Encyclopädie der technischen Chemie, 4. Auflage (1977), Seiten 351 ff). Es wird auch durch den Einsatz von Schleifenreaktoren eine Kreislauffahrweise realisiert, um genügend Verweilzeit für einen vollständigen Umsatz bei begrenztem Reaktionsvolumen (bzw. Holdup) zu schaffen.

Die Isocyanatsynthese erfolgt häufig in der ersten Stufe bei sehr tiefer und in der zweiten bei deutlich höherer Temperatur in einem Verweilzeitapparat. Diese Verfahrensweise wird häufig als Kalt-Heiß-Phosgenierung bezeichnet und ist beispielsweise in W. Siefken, Liebigs Analen der Chemie 562 (1949), Seite 96, beschrieben. Zuerst wird bei tiefer Temperatur, insbesondere bei 0°C oder Raumtemperatur, aber maximal 60°C eine Suspension der Zwischenprodukte Carbamoylchlorid und Aminhydrochlorid hergestellt, die dann bei höheren Temperaturen, insbesondere im Bereich zwischen 100-200°C, in einem Verweilzeitapparat zum Isocyanat umgesetzt wird. Solche zweistufigen Verfahren werden in Kunststoffhandbuch, Band 7 (Polyurethane), 3. neubearbeitete Auflage, Carl Hanser Verlag, München-Wien, S. 76ff (1993), und beispielsweise in den Patentschriften DE 2058032, DE 2153268, DE 2908703, DE 1233854, beschrieben.

In DE 949227 wird ein Kalt-Heißphosgenierverfahren zur kontinuierlichen Herstellung von Isocyanaten durch Umsetzung von Aminen mit Phosgen in flüssiger Phase in Gegenwart eines Lösungsmittel beschrieben, dadurch gekennzeichnet, daß in der Kaltphosgenierung eine Lösung oder Aufschlämmung des Amins in einem inerten Lösungsmittel mit flüssigen Phosgen oder einer Lösung von Phosgen in einem inerten Lösungsmittel kontinuierlich und ohne Außenkühlung in einer Mischvorrichtung unter intensivem Rühren zusammengebracht und das so erhaltenen Reaktionsgemisch dann der Heiß-phosgenierung unterworfen wird. Als Mischeinrichtung werden Turbomischer und Kreiselpumpe und generell Mischeinrichtungen mit mechanisch bewegten Teilen beansprucht. Die Verweilzeit in der Mischeinrichtung beträgt wenige Sekunden bis eine Minute.

In DE 949228 wird ein Kalt-Heißphosgenierverfahren zur kontinuierlichen Herstellung von einkernigen aromatischen Diisocyanaten (z.B. Toluylendiisocyanat) beschrieben, wobei man kontinuierlich aus dem dem Isocyanat zugrundeliegenden Amin und der Gesamtmenge des bei der Phosgenierung verwendeten Lösungsmittels eine Suspension herstellt, diese laufend mit Phosgen in der Kälte umsetzt, das Reaktionsprodukt anschließend kontinuierlich durch ein oder mehrere senkrecht oder schräg stehende Rohre, sogenannte Phosgeniertürme, in denen das Material, gegebenenfalls unter Zufuhr von gasförmigem Phosgen, auf die Phosgenierungstemperatur aufgeheizt wird, hindurchdrückt und anschließend die Lösung durch Begasen mit einem trockenen Inertgasstrom in einer Kolonne von gelöstem Phosgen befreit. Als Lösungsmittel wurde o-Dichlorbenzol verwendet. Die Kaltphosgenierung wird bei 0°C in einem Rührkessel durchgeführt, dann wird die Reaktionsmischung auf 30°C vorerwärmt, und schließlich bei 170°C in zwei in Serie hintereinandergeschalteten Phosgeniertürmen in der Heißphosgenierung zum Isocyanat durchreagiert. Am Fuß des zweiten Phosgenierturmes wird gasförmiges Phosgen eingeleitet. Das in den beiden Phosgeniertürmen über Kopf abgezogene Chlorwasserstoff/Phosgen/Lösungsmittelgemisch wird in dem jeweiligen Kopfkondensator des jeweiligen Phosgenierturms partiell kondensiert und in den Sumpf zurückgeführt. Der nicht-kondensierbare Anteil aus Phosgen und Chlorwasserstoff wird einer Phosgen/Chlorwasserstoff-Trennanlage oder der Vernichtung zugeführt. Das den zweiten Phosgenierturm verlassende flüssige und vollständig durchphosgenierte Reaktionsprodukt läuft in eine Glockenbodenkolonne und wird dort durch Strippen mit einem Stickstoffstrom im Gegenstrom vom gelösten Phosgen befreit. Die am Kopf abgezogenen Brüden werden einem Kondensator zugeführt, und auskondensiertes Lösungsmittel wird auf den Kopf der Kolonne zurückgeführt. Am Sumpf der Kolonne wird das Reaktionsprodukt im Lösungsmittel abgezogen und der Destillation zugeführt.

Auch in DE 952086 wird eine Kalt-Heißphosgenierung beschrieben. Die Heißphosgenierung wird in senkrecht stehenden, mit Raschig-Ringen oder anderen Füllkörpern gefüllten Reaktionstürmen durchgeführt. Die Kaltphosgenierung wird bei 0°C und die Heißphosgenierung mit einem ansteigenden Temperaturprofil von 120°C auf 160°C durchgeführt.

In DE 958558 wird das im Kreislauf geführte Lösungsmittel nicht am unteren Ende der Reaktionstürme, sondern der Kaltphosgenierstufe zugeleitet. Die Verdünnung des Reaktionsproduktes der Kaltphosgenierung bringt den Vorteil einer Lösung mit geringerer Viskosität anstelle einer zähen Suspension von Carbamoylchlorid und Aminhydrochlorid.

Auch in DE 2058032 wird eine Kalt-Heißphosgnierung beschrieben. Die Heißphosgenierung wird in waagerecht liegenden Rohrreaktoren durchgeführt bei Temperaturen von bis zu etwa 200°C, dadurch gekennzeichnet, daß das Reaktionsgemisch aus der Kaltphosgenierung in der Stufe der Heißphosgenierung unter ständiger mechanischer Durchmischung mit langsam ansteigendem Temperaturprofil ausreagiert wird. Die Kaltphosgenierung erfolgt bei einer Temperatur von 0°C.

In US 2908703 wird ein zweistufiges Verfahren zur Herstellung aromatischer Isocyanate beschrieben, in dem der erste Reaktionsschritt bei einer Temperatur von 60-90°C, bevorzugt 70-85°C, mit Chlorbenzol als Lösungsmittel, und der zweite Reaktionsschritt bei einer Temperatur durchgeführt wird, die hoch genug ist, um das Zwischenprodukt zum Isocyanat zu zersetzen. Eine Lösung von Amin in einem organischen Lösungsmittel, bevorzugt Chlorbenzol oder o-Dichlorbenzol, und gasförmiges Phosgen werden gleichzeitig in einen gerührten und beheizten Reaktor gegeben, so daß eine gesättigte Phosgenlösung entsteht und der Phosgenüberschuß mindestens 50 % zur stöchiometrischen Phosgenmenge für die Amin-Phosgen-Reaktion beträgt. Anschließend wird die Reaktionsmischung auf die Zersetzungstemperatur des Carbamoylchlorids und Aminhydrochlorids erhitzt. Das gebildete Isocyanat wird schließlich durch fraktionierte Destillation oder andere Methoden abgetrennt. Alternativ zu dieser Batch-Fahrweise kann das beschriebene Verfahren auch kontinuierlich durchgeführt werden, indem der erste Reaktionsschritt in einem ersten und der zweite in einem zweiten Reaktor durchgeführt wird. Die Reaktion wird in der Regel bei Normaldruck durchgeführt. Die Konzentration des Amins im organischen Lösungsmittel beträgt 2-20 Gew.-%, bevorzugt 5-10 Gew.-%. Höhere Konzentrationen führen zur Bildung von Nebenprodukten, insbesondere Harnstoffen und Polyharnstoffen.

In US 3381025 wird die Phosgenierung eines organischen primären Amins zum entsprechenden Isocyanat zweistufig bei einer Temperatur <60°C in der ersten Stufe und von 100-190°C in der zweiten Stufe durchgeführt. Aus der zweiten Reaktionsstufe wird eine Mischung aus dem inerten Lösungsmittel, überschüssigem Phosgen und dem gebildeten Chlorwasserstoff abgezogen und der Chlorwasserstoff durch Abkühlen der Mischung auf -20°C von dieser abgetrennt. Die erhaltene flüssige kalte Mischung aus Phosgen und Lösungsmittel wird in die erste Reaktionsstufe zurückgeführt.

In DE 2153268 wird ein Verfahren zu einer kontinuierlichen Kaltphosgenierung von organischen primären Aminen durch Umsetzung mit einer Lösung von Phosgen in einem inerten Lösungsmittel in einer mehrstufigen, nicht selbstansaugenden Kreiselpumpe beschrieben. Die Kreiselpumpe fördert gleichzeitig das so erhaltene Reaktionsgemisch in die nachfolgende Heißphosgenierstufe. Die Eintrittstemperatur in die Pumpe beträgt -105°C bis +25°C für die Phosgenlösung und 50 bis 100°C für die Aminlösung. Die Austrittstemperatur des Reaktionsgemisches ist 50 bis 110°C. Die Konzentration der Aminlösung beträgt 5 bis 40 Gew.-%, die der Phosgenlösung 20-65 Gew.-%. Die Phosgenmenge beträgt mindestens 1 mol, bevorzugt 1,5-3 mol, pro mol Amingruppe.

Nachteilig bei der zweistufige Fahrweise mit tiefer Temperatur in der ersten und hoher Temperatur in der zweiten Stufe (Kalt-Heiß-phosgenierung) sind die geringen Reaktionsgeschwindigkeiten und damit geringe Raum-Zeit-Ausbeuten aufgrund der niedrigen Temperaturen in der ersten Phosgenierstufe. Die tiefen Temperaturen (hohe Löslichkeit von Phosgen) und die langen Reaktionszeiten (große Reaktoren) impliziert außerdem einen hohen Phosgen-Holdup, was sicherheitstechnisch unerwünscht ist. Niedrige Temperaturen sind auch wegen des massiven Ausfalls des intermediär gebildeten und bei erhöhten Temperaturen schnell zerfallenden Carbamoylchlorids problematisch. Dies birgt die Gefahr der Verstopfung und der Anbackungen. Desweiteren ist die Abkühlung der Reaktanden und spätere Erwärmung der Reaktionsmischung energetisch nachteilig. Zur Erzielung wirtschaftlicher Raum-Zeit-Ausbeuten ist das Arbeiten bei erhöhter Temperatur in allen Stufen in großtechnischen Verfahren zur Herstellung organischer Isocyanate durch Phosgenierung primärer organischer Amine erforderlich. Bei hohen Temperaturen verringert sich allerdings die Löslichkeit des Phosgens im Reaktionsgemisch und damit der zur Reaktion zur Verfügung stehende Phosgenüberschuß, da die Reaktion in der Regel in der Flüssigphase stattfindet. Ein hoher Phosgenüberschuß ist aber erforderlich, um hohe Ausbeuten an Isocyanat zu erzielen. In EP 0716079 werden der Einfluß von Druck und Temperatur auf die Reaktion und den Phosgenüberschuß beschrieben. Der Erniedrigung des Phosgenüberschusses bei höheren Temperaturen wird im allgemeinen durch einen erhöhten Reaktionsdruck begegnet. In DE-OS 1768439 wird ein Verfahren zur kontinuierlichen Herstellung organischer Isocyanate beschrieben, das durch die Kombination von hoher Temperatur oberhalb 180°C und hohem Druck von 20 bis 150 atm sowie hoher Phosgenkonzentration im Umsetzungsbereich gekennzeichnet ist. Die Menge des eingeführten Amins beträgt das 2,5-fache bis 5,5-fache der stöchiometrischen Menge. Durch den überaus hohen Druck und die sehr hohe Temperatur können noch akzeptable Raum-Zeit-Ausbeuten erzielt werden. Die Verweilzeit der Reaktanden in der Reaktionszone beträgt 5-60 s. Das bevorzugte Lösungsmittel ist Chlorbenzol. Der Nachteil des Verfahrens sind die verminderte Ausbeute und Qualität aufgrund der durch die hohe Temperatur verstärkten Bildung von Nebenprodukten, insbesondere von Harnstoffen. Außerdem sind großtechnische Druckapparaturen sehr teuer und wegen der hohen Giftigkeit des Phosgens problematisch. Die Durchführung der Reaktion zwischen Amin und Phosgen bei hohem Druck hat auch den Nachteil, daß gemäß dem Henry'schen Gesetz neben der Phosgen- auch die Chlorwasserstoff-Konzentration in der flüssigen Phase erhöht wird. Die verstärkte Bildung von Aminhydrochloriden ist aber unerwünscht, da deren Phosgenierung nach gültiger Lehrmeinung sehr langsam ist und daher den geschwindigkeitsbestimmenden Schritt der Gesamtreaktion darstellt. Dies führt zu sehr langen Verweilzeiten und Phosgen-Holdups.

In EP 0065727 wird ein Verfahren mit Düse und Rohrreaktor beschrieben. Es wird ein Verfahren zur kontinuierlichen Herstellung von organischen Mono- und Polyisocyanaten in einer einstufigen Reaktion durch kontinuierliche Vereinigung von Lösungen primärer Mono- oder Polyamine in inerten organischen Lösungsmitteln mit überschüssigen Mengen an in einem inerten organischen Lösungsmittel gelösten Phosgen bei Drücken von 10 bis 1000 bar, bevorzugt 25 bis 150 bar, und Temperaturen von 120 bis 300°C, bevorzugt 150 bis 250°C in einem Mischraum und gegebenenfalls einer nachgeschalteten Reaktionszone mit kontinuierlicher Aufarbeitung beschrieben, wobei die im Überschuß eingesetzte Phosgenlösung kontinuierlich in einem Mischraum vorgelegt und die im Unterschuß eingesetzte Aminkomponente mittels einer Glattstrahldüse eingedüst wird. Die für das Verfahren wesentliche Glattstrahldüse hat einen lichten Durchmesser von 0,1-30 mm.. Es wird ein Differenzdruck von mindestens 0,5 bar, vorzugsweise 1-200 bar, insbesondere 3-50 bar in die eingedüste Phosgenlösung eingehalten. Die Molverhältnisse von Phosgen zu Aminogruppen betragen 2:1 bis 30:1, bevorzugt 3:1 bis 18:1. Die Nachreaktionszone kann ein Rohrreaktor, ein Rohrbündelreaktor oder beispielsweise eine Rührkesselkaskade sein. Die mittlere Verweilzeit im Mischgefäß und in der nachgeschalteten Reaktionszone beträgt zwischen 5 Sekunden und 5 Minuten. Das die Nachreaktionszone verlassende Reaktionsgemisch wird ein- oder mehrstufig auf Normaldruck in ein Entspannungsgefäß entspannt, wodurch ein Temperaturabfall von 50-150°C auftritt. Im Entspannungsgefäß werden Gas- und Flüssigphase getrennt. Als bevorzugte Lösungsmittel werden Chlorbenzol oder o-Dichlorbenzol eingesetzt.

In GB 827376 wird ein kontinuierliches Verfahren zur Herstellung aromatischer Isocyanate durch Reaktion eines Amins in freier Form in einem Lösungsmittel oder als in diesem Lösungsmittel suspendiertes, leicht zum Amin zu zersetzendes Salz und einer Lösung von Phosgen in einem inerten Lösungsmittel bei einem Druck von größer als 3*10⁵Pa, wobei die Reaktanden unter Vermischung gleichzeitig eingeführt werden in das untere Ende eines vertikalen Rohrreaktors, in dem die Reaktionsprodukte schnell zum oberen Ende aufsteigen, beschrieben. Die Flüssigphase wird in einem Behälter aufgefangen, aus dem sie zur Isolierung des Isocyanats abgenommen wird. Dieser Behälter kann ein Phasentrennapparat sein, der unter dem gleichen Druck betrieben wird, über ein Überlaufrohr mit dem Flüssigauslaß kommuniziert und ein Drosselventil im Flüssigauslaß besitzt. Die im Behälter abgetrennte Flüssigkeit wird einer Kolonne zugeführt, die unter Atmosphären- oder Überdruck und erhöhter Temperatur betrieben wird, wodurch restliches Phosgen und Chlorwasserstoff gasförmig über Kopf abgetrennt werden. Aus der im Behälter abgetrennten Chlorwasserstoff/Phosgenmischung wird das überschüssige Phosgen auskondensiert (vorzugsweise mit Kühlwasser), und der so abgetrennte Chlorwasserstoff entspannt und abgeführt. Die Reaktanten werden mit einer gemeinsamen oder zwei getrennten Pumpen dem Rohreaktor zugeführt oder aber in einer Venturi-Mischdüse, vorzugsweise mit getrennten Einlässen für die beiden Reaktanten, vermischt und von dort in den Rohrreaktor eingebracht. Die beschriebene Temperatur im Rohrreaktor beträgt 80-200°C, der Druck ist größer 3*10⁵ Pa, maximal dem Dampfdruck der Reaktionsmischung und vorzugsweise 15-20*10⁵ Pa.

In US 3226410 wird ein kontinuierliches Verfahren zur Herstellung aromatischer Isocyanate durch Einmischen eines Stromes eines aromatischen Amins in einen Phosgenstrom in einem Rohrreaktor bei Reynoldszahlen von größer 2100 (bevorzugt 5000-2000000) und Temperaturen von 60-90°C, bevorzugt 80-85°C, beschrieben. Die Phosgenmenge beträgt mindestens 1 mol, bevorzugt 6 bis 12 mol, pro mol Amin. Die Reaktionslösung wird dann, gegebenenfalls nach Vorerwärmung, in einen zweiten Reaktor, insbesondere ein Kessel oder eine Kolonne, überführt, der eine Temperatur von 110 bis 135°C, bevorzugt 110 bis 120°C, besitzt. Die Aminkonzentration beträgt 2 bis 25 Gew.-%, bevorzugt 5 bis 10 Gew.-%, die Phosgenkonzentration 10 bis 100 Gew.-%, bevorzugt 10 bis 60 Gew.-%. Der Druck, mit dem der Phosgenstrom in den Rohrreaktor überführt wird, beträgt 50-170 psig; der Druck des Aminstroms muß größer sein, um Rückvermischung zu verhindern. Aus dem zweiten Reaktor wird die Flüssigphase, enthaltend Isocyanat, Lösungsmittel, relativ geringe Mengen Nebenprodukte, Chlorwasserstoff und Phosgen gelöst im Lösungsmittel, getrennt von der Gasphase, enthaltend Chlorwasserstoff, Lösungsmittel, Phosgen und Spuren des Isocyanats, abgezogen. Als Lösungsmittel werden chlorierte Kohlenwasserstoffe, die inert sind und einen geringeren Siedepunkt als das Isocyanat besitzen, verwendet. Besonders bevorzugt ist Chlorbenzol.

An den zweiten Reaktor mit einem Druck von 45 psig oder höher schließen sich ein Verweilzeitbehälter und ein Puffergefäß an, aus dem standgeregelt in eine Kolonne zum Entfernen von überschüssigem Phosgen gefahren wird. Phosgen, Chlorwasserstoff und Lösungsmittel werden über Kopf genommen und in den Phosgenbehälter zurückgeführt. Das Sumpfprodukt, bestehend aus Isocyanat und Lösungsmittel wird in die destillative Lösungsmittelabtrennung vorzugsweise einstufig gefahren. Das vom Isocyanat abgetrennte Lösungsmittel wird zur Absorption des restlichen Phosgens aus dem Chlorwasserstoffstrom verwendet. Das im zweiten Reaktor und im Pufferbehälter abgezogene Phosgen wird zweistufig kondensiert und in den Phosgenbehälter zurückgeführt. Das nichtkondensierte Phosgen/Chlorwasserstoff-Gemisch wird in einen Phosgenabsorber gefahren, der mit in der Lösungsmittelabtrennung gewonnenem Lösungsmittel beaufschlagt wird. Das nicht absorbierte Gas, hauptsächlich Chlorwasserstoff, wird anschließend in einem Absorber mit Wasser zu wässriger Salzsäure umgesetzt.

Der Rohrreaktor soll als "Plugflow"-Reaktor ohne Umlenkungen, Taschen oder andere Einbauten, die Totzonen erzeugen können, konstruiert sein, um das Absetzen von Feststoffen zu verhindern. Die hohen Renoldszahlen und das Design des Reaktors als gerade Röhre sollen dazu führen, daß die Flüssigkeit ständig die Wandungen von Anbackungen freispült.

In DE 952086 wird ein Verfahren zur Herstellung von Isocyanaten aus primären Aminen bzw. deren Salzen und Phosgen beschrieben, dadurch gekennzeichnet, daß man die Reaktionspartner bei der Heißphosgenierung in Gegenwart eines Lösungs- bzw. Verdünnungsmittels kontinuierlich von unten her durch ein aufrecht oder schräg stehendes beheiztes Rohr leitet. Nach dem Passieren dieses ersten Reaktionsrohres kann sich gegebenenfalls zur Vervollständigung der Reaktion unter Zusatz von weiterem Phosgen ein zweiter, ebenfalls aufrecht stehender Reaktionsturm anschließen. Der Vorteil von senkrecht stehenden Reaktionstürmen, die mit Raschig-Ringen oder anderen Füllkörpern zur Verhinderung eines raschen Ausgasens gefüllt sind, ist, daß die Geschwindigkeit der Isocyanatbildung erhöht wird aufgrund der höheren Phosgenkonzentration an der unten liegenden Eintrittsstelle der Reaktanden, die sich durch den hydrostatischen Druck der Flüssigkeitssäule einstellt. Als Lösungsmittel wird o-Dichlorbenzol genannt. Das Verfahren ist ein zweistufiges Verfahren mit einer Kaltphosgenierung als erste Stufe und einer Heißphosgenierung als zweite Stufe. Die Kaltphosgenierung wird bei 0°C und die Heißphosgenierung mit einem ansteigenden Temperaturprofil von 120°C auf 160°C durchgeführt.

Nachteilig an diesem Verfahren sind zum einen die grundlegenden Schwachstellen der Kalt-/Heißphosgenierung und zum anderen der geringe erreichbare Druck. Durch Druckbehälter und Regelventile können erheblich höhere Drücke und damit Phosgenkonzentrationen in der Flüssigphase eingestellt werden. Nachteilig sind außerdem die in den Rohreaktoren verwendeten Füllkörper, da die bei der Kaltphosgenierung gebildeten und ausgefallenen festen Zwischenprodukte Carbamoylchlorid und Aminhydrochlorid leicht zur Verstopfung und damit zu geringer Verfügbarkeit der Anlage führen können.

In DE 2058032 wird ein Verfahren zur Herstellung von Isocyanaten aus Aminen und Phosgen in Gegenwart eines inerten Lösungsmittels beschrieben, wobei das Reaktionsgemisch in der Kaltphosgenierung bei Temperaturen von 0°C behandelt und anschließend in der Heiß-phosgenierung bei Temperaturen von bis zu etwa 200°C ausreagiert wird, wobei das Reaktionsgemisch aus der Kaltphosgenierung in der Stufe der Heißphosgenierung unter ständiger mechanischer Durchmischung mit langsam ansteigendem Temperaturprofil durch einen sich waagerecht erstreckenden Reaktionsraum geleitet wird. Als beheizbares Reaktionsgefäß für die Heißphosgenierung wird ein waagerecht liegendes Rohr beansprucht, durch welches in Längsrichtung eine mit Rohreinrichtungen besetzte Welle erstreckt und welches separat beheizbare Abschnitte einer Mantelheizung besitzt. Am Ende des Rohrreaktors kann ein Naturumaufverdampfer (Thermosyphon) angeordnet sein. Es wird auch eine Anordnung von drei U-förmig miteinander verbundenen Reaktionsrohren beschrieben (System kommunizierender Röhren). Im ersten, senkrecht stehenden Rohr wird die Kaltphosgenierung, im waagerechten die Heißphosgenierung und im zweiten senkrechten Rohr die Entgasung, das heißt die Entfernung von Chlorwasserstoff und Phosgen aus dem Reaktionsgemisch, durchgeführt. Bei einem solchen System kann auf Förderpumpen und Regelventile verzichtet werden, da die Produktströmung sich nach dem Zulauf einstellt.

In DE 2747524 wird ein kontinuierliches Verfahren zur Herstellung aromatischer Isocyanate beschrieben, bei dem dem Reaktor soviel Wärme zugeführt wird, daß zugesetztes Phosgen nicht zu einer Abkühlung und damit verbunden zu Anbackungen des Zwischenproduktes Carbamoylchlorid auf der Reaktorwand führt. Es wird ein Pfropfenflußreaktor beschrieben, der aus zwei koaxialen Rohren besteht, in die die beiden Reaktanden Amin und Phosgen in einem inerten organischen Lösungsmittel jeweils isoliert voneinander im Gegenstrom eingeführt und am Ende des inneren Rohres vermischt werden. Die Rückvermischung in die Beschickungszone sei ausgeschaltet, was die Nebenproduktbildung minimiere. Die Temperierung mit einem Wasserdampfmantel erfolgt, um ein Verstopfen der Mischzone mit dem Zwischenprodukt Carbamoylchlorid zu verhindern. Es seien Temperaturen von 90-140°C erforderlich, im allgemeinen werden Temperaturen von 90-200°C angegeben. Die Anfangstemperatur liegt allerdings bei 60-90°C. Praktische Betrachtungen bestimmen die Obergrenze der Temperatur. Als bequemer Druck wird 2 atü angegeben. Als Aminkonzentration im inerten Lösungsmittel werden 2 bis 20 %, bevorzugt 5 bis 10 % genannt. Als inertes Lösungsmittel wird Dichlorbenzol bevorzugt.

Ein Rohrreaktor ist auch der bevorzugte Apparat in dem in WO 96/16028 beschriebenen Verfahren zur Herstellung von Isocyanaten mit Isocyanat als Lösungsmittel. In WO 96/16028 wird ein kontinuierliches, einstufiges Verfahren beschrieben, in dem das gegebenenfalls in einem inerten, organischen Lösungsmittel gelöste primäre Amin mit Phosgen, das im Isocyanat zu 10-60 Gew.-%, bezogen auf die Isocyanat/Phosgen-Lösung, gelöst ist, bei Temperaturen von 60-180°C und Drücken von 1-30 bar zum entsprechenden Isocyanat umgesetzt wird, wobei das molare Verhältnis von Phosgen zu eingesetztem Amin 4:1 bis 1:1 beträgt und das als Lösungsmittel eingesetzte Isocyanat feststofffrei ist und einen Wert für hydrolysierbares Chlor von kleiner 2 % besitzt.

In DE 19817691 wird ein zweistufiges Verfahren zur Herstellung von Mischungen aus Diphenylmethandiisocyanaten (MDI) und Polyphenylen-polymethylen-polyisocyanaten (PMDI) mit vermindertem Gehalt an chlorierten Nebenprodukten und verminderter Jodfarbzahl durch zweistufige Umsetzung der entsprechenden Mischungen aus Diphenylmethan-diaminen (MDA) und Polyphenylen-polymethylendiaminen (PMDA) mit Phosgen in Gegenwart mindestens eines organischen Lösungsmittels bei erhöhter Temperatur, nach beendeter Phosgenierung Abtrennung des überschüssigen Phosgens und Lösungsmittels und thermische Behandlung des Reaktionsproduktes, wobei die Molverhältnisse von Phosgen zu Chlorwasserstoff im Verweilzeitapparat der zweiten Stufe der Phosgenierung gleichzeitig in der Flüssigphase 10 bis 30:1 und in der Gasphase 1 bis 10:1 betragen beschrieben. Die in der ersten Stufe der Phosgenierung, einem statischen Mischer, gebildeten Carbamoylchloride und Aminhydrochloride durchlaufen in der zweiten Stufe der Phosgenierung einen Verweilzeitapparat, in dem die Aminhydrochloride zu den entsprechenden Carbamoylchoriden phosgeniert und die Carbamoychloride zu den entsprechenden Isocyanaten und Chlorwasserstoff gespalten werden. Die Temperatur der ersten Stufe beträgt üblicherweise 40 bis 150°C, bevorzugt 60 bis 130°C, besonders bevorzugt 90 bis 120°C. Als statische Mischer für die erste Stufe kommen insbesondere Düsen zur Anwendung. Als Verweilzeitapparat für die zweite Stufe wird neben Rührmaschinen, Rührkesselkaskaden besonders bevorzugt eine Kolonne, insbesondere eine Reaktionskolonne mit vorwiegend < 10 theoretischen Böden verwendet. Hierbei ist es besonders vorteilhaft, diese im Gegenstrom zu betreiben. Die Sumpftemperatur der Kolonne beträgt vorzugsweise 80 bis 120°C, besonders bevorzugt 90 bis 110°C. Der Kopfdruck der Kolonne beträgt vorzugsweise 1,0 bis 4,7 at (Ü), besonders bevorzugt 2,0 bis 3,7 at (Ü).

Nachteilig an diesem Verfahren ist, daß die Aminhydrochloridphosgenierung und der Carbamoylchloridzerfall in ein und demselben Reaktor durchgeführt werden, was zu längeren Verweilzeiten und höheren Phosgen-Holdups führt.

In US 3544611 wird ein Verfahren zur Herstellung von organischen Isocyanaten bei hohem Druck von 10 bis 50 bar beschrieben. Überraschenderweise wurde gefunden, daß die Durchführung der Reaktion bei höheren Drücken von mindestens 10 atü zu höheren Ausbeuten an Isocyanat führt. Des weiteren wird durch höhere Drücke die Chlorwasserstoff/Phosgentrennung erleichtert. Der erste Reaktionsschritt der Isocyanatherstellung, die Reaktion zwischen Amin und Phosgen zum Zwischenprodukt Carbamoylchlorid, wird in einem Schleifenreaktor (Mischkreis) durchgeführt. Der zweite Reaktionsschritt, die Zersetzung des Carbamoylchlorids zum Isocyanat, erfolgt in einer dem Mischkreis nachgeschalteten Reaktionskolonne, wobei am Kopf der Kolonne eine Chlorwasserstoff/Phosgenmischung anfällt. Daraus wird Phosgen zweistufig auskondensiert. Das auskondensierte Phosgen wird auf den Kopf der Kolonne zurückgeführt. An einem Flüssigabzug im Verstärkungsteil der Kolonne wird Phosgen entnommen und der Reaktion (dem Mischkreis) wieder zugeführt.

Die Restphosgenabtrennung aus der Reaktionsmischung, welche am Sumpf der Reaktionskolonne entnommen wird, erfolgt in einer weiteren Kolonne. In letzterer wird Phosgen über Kopf genommen, analog zur ersten Kolonne zweistufig kondensiert und in den Mischkreis zur Reaktion zurückgeführt. Die Reaktion zum Isocyanat wird in der Reaktionskolonne abgeschlossen.

In DE 3736988 wird ein kontinuierliches Verfahren zur Herstellung von organischen Mono- oder Polyisocyanaten in einstufiger Reaktion durch Umsetzung von dem in einem organischen Lösungsmittel gelösten Amin mit in einem organischen Lösungsmittel gelöstem Phosgen in einer Reaktionskolonne bei einer Temperatur unterhalb von 150°C beschrieben. Das Reaktionsgemisch läßt man kontinuierlich von unten nach oben die Reaktionskolonne durchlaufen. Die Reaktionskolonne besitzt mindestens 10, durch Lochböden voneinander getrennte Kammern. Die Konzentration des Amins im inerten Lösungsmittel beträgt 5-40 Gew.-%, vorzugsweise 7-20 Gew.-%. Bevorzugte Lösungsmittel sind Chlorbenzol oder Dichlorbenzol oder Mischungen davon. Phosgen wird als 30-65 Gew.-%, bevorzugt 40-65 Gew.-% Lösung im inerten Lösungsmittel eingesetzt. Das Äquivalentverhältnis von Amin zu Phosgen beträgt 1:1,5 bis 1:7, vorzugsweise 1:2 bis 1:5. Die Temperatur am Kopf der Kolonne beträgt bevorzugt 70-130°C, besonders bevorzugt 90-125°C, maximal 150°C. Die mittlere Verweilzeit in der Reaktionskolonne beträgt maximal 120 Minuten, vorzugsweise maximal 60 Minuten. Der Druck in der Kolonne beträgt 1,2-3 bar abs, vorzugsweise 1,5-2,5 bar abs.

In DE 3744001 wird als Verweilzeitapparat ebenfalls eine von unten nach oben durchströmte Lochbodenkolonne mit > 10 Lochböden, bevorzugt 20-50 Lochböden, einer Verweilzeit von max. 120 Minuten, vorzugsweise maximal 60 Minuten, und Flüssiggeschwindigkeiten von 0,05-0,4 m/s, vorzugsweise 0,1-0,4 m/s, und Gasgeschwindigkeiten von 2-20 m/s, vorzugsweise 3,5-10 m/s, vorgeschlagen. Durch die horizontal eingebauten Lochböden werden 10-50 Kammern gebildet. Die Kopftemperatur der Reaktionskolonne liegt unterhalb von 150°C, bevorzugt bei 70-130°C, besonders bevorzugt bei 90-125°C. Der Kopfdruck der Kolonne beträgt 1,2-3 bar (abs.), bevorzugt 1,5-2,5 bar (abs.). Für die erste Phosgenierstufe wird eine Mischdüse beansprucht.

In EP 0291819 wird ein zweistufiges Verfahren zur Herstellung von Isocyanaten nach der Kalt-Heißphosgenierung beschrieben, wobei für die Kaltphosgenierung ein Mischer mit Rotorscheibe eingesetzt wird, und wobei für die Heißphosgenierung bevorzugt Phosgeniertürme verwendet werden. Die Phosgeniertürme werden bei Normaldruck oder leichtem Überdruck von bis zu 1,5 atü betrieben. Besonders vorteilhaft ist es, das die Mischvorrichtung verlassende Reaktionsgemisch der Kaltphosgenierung kontinuierlich von oben oder unten in einen beheizbaren Turm eintreten zu lassen und die Reaktion durch Zuführung von Wärme zu vollenden. Zur Einstellung eines bestimmten Temperaturprofils können auch mehrere Türme hintereinander geschaltet oder eine Kombination von Türmen und Kesseln verwendet werden.

In DE 2112181 (US 3829458) werden organische Isocyanate aus primären organischen Aminen und Phosgen in einem inerten organischen Lösungsmittel kontinuierlich in einem oder mehreren, Füllkörper enthaltenden Reaktionsgefäßen, die im Gleichstrom durchflossen werden, in der sogenannten Übergangsströmung hergestellt. Die Übergangsströmung besteht aus einer aminhaltigen, flüssigen organischen Phase und einer phosgenhaltigen Gasphase. Die Umsetzung findet bei Reaktionstemperaturen zwischen 50 und 220°C statt. Bei nicht vollständigem Umsatz wird die Reaktionsmischung mehrfach im Kreislauf durch die mit Füllkörpern gefüllte Säule geführt. Nachteilig an diesem Verfahren ist die hohe Anfälligkeit für Verstopfung der Füllkörperkolonne, die durch sich auf den Füllkörpern ablagernde Feststoffe wie Carbamoylchlorid, Aminhydrochlorid, Harnstoffe u.a. verursacht wird. Des weiteren sind Füllkörperkolonnen durch einen hohen Druckverlust gekennzeichnet, was hohe Sumpftemperaturen und damit eine starke thermische Belastung der Reaktionsmischung und des gebildeten Isocyanats mit der Folge erhöhter Nebenproduktbildung und reduzierter Ausbeute bedingt.

In vielen Verfahren wird die Reaktion von Phosgen und Amin in einem Schleifenreaktor oder Kreislaufreaktor durchgeführt, in den neben den Feedströmen aus Amin und Phosgen, gegebenenfalls in einem Lösungsmittel, zumindest ein Teil der Reaktionsmischung rezykliert wird. Diese Verdünnung durch Rückführung der gebildeten Reaktionsmischung dient hauptsächlich der Temperaturkontrolle bzw. der besseren Wärmeabfuhr zur Einstellung tiefer Temperaturen. Die Reaktion zwischen Aminen und Phosgen ist stark exotherm. Bei ungünstiger Reaktionsführung und Apparategestaltung bedingen höhere Temperaturen eine verstärkte Nebenproduktbildung, welche z.B. im Falle von Toluylendiisocyanat (TDI) zu einem Ausbeuteverlust und zu Teeranfall führt. Als Hauptnebenprodukte fallen Harnstoffe an.

In DE 2624285 (BASF) wird ein Mischkreisverfahren zur kontinuierlichen Herstellung von organischen Isocyanaten aus organischen Aminen und Phosgen in Gegenwart von organischen Lösungsmitteln beschrieben, wobei man das Phosgen der im Kreislauf geführten Reaktionslösung zumischt und das erhaltene Reaktionsgemisch und die Amine oder Aminlösung der Misch- und Reaktionszone so zuführt, daß in dieser eine Energiedissipationsdichte von 5 bis 1000 kJ pro m³ rückgeführten Reaktionsgemisches plus zugeführter Aminlösung erzeugt wird. Die Umsetzung erfolgt bei Temperaturen von 90 bis 220°C, vorzugsweise 120 bis 180°C und in einem Druckbereich von 1 bis 10 bar, vorzugsweise 1 bis 3 bar. Die Verweilzeiten betragen 10 bis 180 Minuten. Das Molverhältnis von Amin zu Phosgen wird so bemessen, daß pro Aminogruppe 1 bis 10 mol, vorzugsweise 1,3 bis 4 mol, Phosgen in der Reaktionsmischung vorliegen. Die Ausbeuten sind 88 bis 98 Gew.-%, bezogen auf das eingesetzte Amin.

Das in DE 2624285 beschriebene Mischkreisverfahren wird in EP 0150435 weiterentwickelt. Das Verfahren zur kontinuierlichen Herstellung von organischen Isocyanaten durch Umsetzung von organischen Aminen mit Phosgen in Gegenwart von organischen Lösungsmitteln, wobei Chlorwasserstoff abgetrennt und die Reaktionsmischung teilweise im Kreislauf geführt wird, ist dadurch gekennzeichnet, dass der Chlorwasserstoffgehalt der nach der Chlorwasserstoffabtrennung zur Aminzugabe rückgeführten Reaktionsmischung vor der Aminzugabe gleich oder kleiner 0,5 Gew.-%, vorzugsweise 0,01 bis 0,4 Gew.-%, bezogen auf das Gesamtgewicht der Reaktionsmischung, ist und das Molverhältnis von Phosgen- zu Aminogruppe der organischen Amine 12 bis 200:1 beträgt. Die Umsetzung wird bei Temperaturen von 100-220°C, vorzugsweise 120-180°C und in einem Druckbereich von 5-100 bar, vorzugsweise 15-50 bar durchgeführt.

In DE 3403204 wird ein Verfahren zur kontinuierlichen Herstellung von organischen Isocyanaten, vorzugsweise Polyisocyanaten, durch Umsetzung von organischen Aminen, vorzugsweise Polyaminen, mit Phosgen in Gegenwart von organischen Lösungsmitteln unter Druck, z.B. von 5 bis 100 bar, bei erhöhten Temperaturen, z.B. von 100 bis 220°C, wobei die Reaktionsmischung teilweise im Kreislauf, vorzugsweise nach dem Naturumlaufprinzip, geführt wird, und der Chlorwasserstoffgehalt der Reaktionsmischung vor der Aminzugabe kleiner als 0,5 Gew.-%, bezogen auf das Gesamtgewicht der Reaktionsmischung ist und das Molverhältnis von Phosgen zu Aminogruppe der organischen Amine 12 bis 200:1 beträgt, beschrieben.

In DE 3212510 wird ein Verfahren zur kontinuierlichen Herstellung organischer Isocyanate beschrieben. Das primäre organische Amin wird in einem praktisch dispergierten Zustand mit einem Überschuß an Phosgen bei einem Manometer- oder Überdruck von 10 kg/cm², etwa 10 bar, und einer Temperatur von 60 bis 100°C in Kontakt gebracht, wodurch ein entsprechendes organisches Carbamoylchlorid aus dem organischen Amin und intermediär entstehendem Hydrochlorid gebildet wird. Als Nebenprodukt Chlorwasserstoff gebildet wird. Der Reaktionsumsatz in dieser ersten Stufe ist dergestalt, daß 30 bis 70 % des Carbamyolchlorids in Isocyanat umgewandelt werden. Die Reaktionsmischung wird bei einem Manometer- oder Überdruck von 10 kg/cm² und einer Temperatur von 120 bis 160°C gehalten, wodurch die Umwandlung des Hydrochlorids zum Carbamylchlorid erreicht und die Umwandlung des Carbamoylchlorids zum Isocyanat beendet wird. Die Reaktion findet in einem Kreislaufreaktor (Umlaufleitung) oder in einem tankförmigen Reaktionsgefäß statt. In erstem Fall wird das Phosgen zusammen mit dem Lösungsmittel in einer röhrenförmigen Umlaufleitung umlaufen gelassen und darin das Amin eingemischt (Mischkreis). Die Verweilzeit in der ersten Stufe beträgt 30-120 Minuten und in der zweiten Stufe 10-120 Minuten. Als Lösungsmittel wird ortho-Dichlorbenzol gewählt.

In GB 763535 und DE 1811609 werden ebenfalls Schleifenreaktoren oder Kreislaufreaktoren (Mischkreise als Reaktionssystem) beschrieben. Die Herstellung des organischen Isocyanats erfolgt durch Umsetzung eines Amins mit Phosgen in einer einstufigen kontinuierlichen Reaktion unter Kreislaufführung von Isocyanat, Lösungsmittel und nicht umgesetztem Phosgen. Der ausreichende Druck im in GB 763535 beschriebenen Verfahrens beträgt 5-20 pounds per square inch, die Reaktionstemperatur ist 90-180°C, die TDA-Konzentration im Lösungsmittel ist 5-30 %, der stöchiometrische Phosgenüberschuß ist mindestens 25 %, bevorzugt 70-110 %, und als Lösungsmittel werden chlorierte aromatische Kohlenwasserstoffe, bevorzugt o-Dichlorbenzol, verwendet. In DE 1811609 wird das organische Amin, gegebenenfalls in ortho-Dichlorbenzol oder einem anderen Lösungsmittel, und Phosgen im Überschuß unter hoher Scherbeanspruchung in die umlaufende Reaktionsmischung eingemischt, wodurch aufgrund der Vermischung vorteilhaft von GB 763535 abweichende Bedingungen eingestellt werden können. Der Reaktionsdruck ist vorzugsweise wenigstens 1,8-14*10⁵ Pa, vorzugsweise 4,2*10⁵ Pa oder 3,5*10⁵ Pa. Die Reaktionstemperatur wird mit vorzugsweise 102-130°C und für Toluylendiamin mit vorzugsweise 90-120°C angegeben. Der Phosgenüberschuß beträgt 50-200 %, vorzugsweise 70 %.

In DE 1037444 (US 2822373) wird ein kontinuierliches Verfahren zur Herstellung organischer Isocyanate beschrieben, wobei eine Lösung des organischen Amins in einem inerten Lösungsmittel mit einer Lösung von Phosgen in einem inerten Lösungsmittel in einer Reaktionszone, die einen Überdruck und eine turbulente Strömung aufweist, bei Temperaturen von 90 bis 180°C zur Reaktion gebracht wird. Die Reaktionslösung wird dann in eine Zone niedrigeren Drucks, üblicherweise Atmosphärendruck, entspannt, wobei Chlorwasserstoff und Phosgen als gasförmige Mischung abgeführt werden. Das Isocyanat wird durch Destillation vom Lösungsmittel abgetrennt. Im beschriebenen Verfahren wird die Aminlösung in eine Umpumpkreislaufleitung gegeben, hinter der Reaktionszone durch einen Wärmetauscher aufgeheizt und dann über eine Drosselventil in ein tankförmiges Verweilzeitgefäß entspannt. Aus diesem wird die Reaktionsmischung wieder für den Umpumpkreis entnommen bzw. teilweise zur Lösungsmittelabtrennung und Reingewinnung des Isocyanats ausgeschleust. Von der gasförmig am Reservoir abgezogenen Mischung aus Chlorwasserstoff, überschüssigem Phosgen und Lösungsmittel wird das Lösungsmittel in einem Kondensator auskondeniert und in das Reservoir zurückgeführt. Als bevorzugte Drücke in der Umpumpleitung sind 5-20 pounds per square inch angegeben. Die Aminkonzentration im Lösungsmittel beträgt 5-30 Gew.-%, die Phosgenmenge ist mindestens 1,25 mol pro Aminogruppe des Amins. Als bevorzugtes Lösungsmittel ist ortho-Dichlorbenzol angegeben.

In US 3574695 wird ein verbessertes kontinuierliches Verfahren zur Herstellung organischer Isocyanate beschrieben. Die Verweilzeiten können verkürzt werden, indem das Produkt aus der ersten Reaktionszone in der zweiten mit mindestens 0,75 mol Phosgen pro Äquivalent organischem Amin, welches in der ersten Reaktionszone zugegeben wurde, behandelt wird. Die Verweilzeit in dieser zweiten Reaktionszone beträgt 5-45 Minuten bei einer Temperatur von mindestens 130°C. Aus der zweiten Reaktionszone werden kontinuierlich eine gasförmige Mischung von Chlorwasserstoff und Phosgen und eine flüssige Lösung des organischen Isocyanats im Lösungsmittel abgezogen.

In GB 1034285 wird ein kontinuierliches Verfahren beschrieben zur Herstellung organischer Isocyanate durch Reaktion von Phosgen mit einem primären Polyamin in Gegenwart eines inerten organischen Lösungsmittels, wobei die Reaktanten getrennt von einander in einen Rohreaktor gespeist und dort in Kontakt gebracht werden, und wobei durch diesen Rohrreaktor eine Mischung des gleichen Lösungsmittels, der Reaktionsmischung und von Phosgen rezykliert wird. Als Reaktor kann eine Verschaltung von zwei zylindrischen Behälter, zwischen denen die Reaktionsmischung im Kreis geführt wird, oder ein ringförmiger Rohrreaktor verwendet werden. Die Reaktionsmischung kann mittels Rührern gerührt werden. Die Temperatur im Rohrreaktor beträgt 8-50°C. Der Druck ist Atmosphärendruck oder geringfügig darüber. Die Konzentration des zudosierten primären Polyamins im Lösungsmittel beträgt 2-20 Gew.-%. Die in den Umpumpstrom zugesetzte Phosgenmenge beträgt 5 bis 20 mol Phosgen pro mol Amingruppen in der zugesetzten Polyaminlösung. Als inertes organisches Lösungsmittel wird Chlorbenzol oder ortho-Dichlorbenzol verwendet.

In GB 1212249 wird ein Verfahren zur Herstellung von Isocyanaten beschrieben, in dem in der ersten Stufe ein Überschuß an Phosgen mit einem vorwärtsgerichteten, durchmischten Film des Amins in einem inerten Lösungsmittel zur Reaktion gebracht wird. Ein zylindrisches Rohr wird als geeignet zur Erzeugung dieses Films angesehen.

In JP 57-048954 wird ein Verfahren zur Herstellung organischer Isocyanate beschrieben, in dem die Lösung eines primären Amins kurz vor die Zulaufstelle eines statischen Mischers zugeführt wird, der sich in einem Kreislaufreaktor befindet. Im Kreislaufreaktor zirkuliert eine Lösung von Phosgen in organischem Isocyanat.

Eine Phosgenierung im Schleifenreaktor wird auch in JP 60-10774 beschrieben, in der eine isocyanathaltige Reaktionsmischung umgepumpt wird, allerdings werden hohe Ausbeuten nur bei Aminkonzentrationen von 5-10 % erzielt.

Energetisch nachteilig an den Schleifenreaktor- oder MischkreisVerfahren sind die niedrigen Temperaturen in der ersten Stufe und die hohen Temperaturen in der zweiten Stufe. Da die Reaktion zwischen einem organischen Amin und Phosgen stark exotherm ist, muß im ersten Schritt stark gekühlt werden, um die gewünschte Reaktionstempratur einzuhalten. Die zweite Reaktion, der Zerfall des Carbamyolchlorids zum Isocyanat ist deutlich endotherm, so daß in der zweiten Stufe die Reaktionsmischung wieder aufgeheizt werden muß.

Besonders nachteilig sind allerdings die deutlich geringere chemische Ausbeute gegenüber Verfahren im geraden Durchgang, da im Mischkreis durch die Rückvermischung bereits gebildetes Isocyanat mit Amin zu Harnstoffen reagiert. Um diese Nebenreaktion zurückzudrängen, wird häufig eine geringe maximale stationäre Isocyanat-Konzentration zugelassen, was wiederum geringe Raum-Zeit-Ausbeuten bedeutet.

Bei dem in EP 0716079 beschriebenen Verfahren zur kontinuierlichen Herstellung organischer Isocyanate wird das Reaktionsgemisch bei einer Temperatur von 60-100°C in einer Blasensäule umgewälzt. Das beschriebene Verfahren wird bei leicht erniedrigtem oder leicht erhöhten Druck, im allgemeinen bei 0,5-5 bar, bevorzugt bei 1-3 bar, durchgeführt.

In EP 0570799 wird ein Verfahren beschrieben, in dem die Reaktion zwischen Amin und Phosgen zum Isocyanat in der Gasphase durchgeführt wird. Die Gasphasenphosgenierung ist zur Herstellung von aliphatischen Diisocyanaten (EP 0289840), aromatischen Diisocyanaten (EP 0570799), cyclischen Diisocyanaten (EP 1078918) und von Triisocyanaten (EP 0749958) bekannt. In EP 0749958, EP 0676392 und EP 0289840 werden Verfahren zur Herstellung aliphatischer Di- und Triisocyanate durch Gasphasenphosgenierung beschrieben, in denen die Vermischung der Rektanden durch am Eintritt in den beschriebenen Rohreaktor durch Düsen oder eine Kombination aus Düse und Ringspalt zwischen Düse und Rohr erfolgt. Für die Vermischung wird hier als wesentliches Kriterium eine Reynoldszahl von RE > 4700 im Rohr angegeben. Für die Herstellung aromatischer Diisocyanate durch Gasphasenphosgenierung wird in EP 0570799 ein Strahlmischer angegeben.

In DE 1192641 wird ein Verfahren zur Herstellung von Isocyanaten durch Umsetzung von primären aromatischen oder araliphatischen Aminen mit Phosgen in Gegenwart von Lösungsmitteln und anschließendes Erhitzen des Umsetzungsgemisches beschrieben, wobei man das Isocyanat als Lösungsmittel verwendet, was in der Umsetzung hergestellt werden soll.

In DE 100 27 779 wird ein Verfahren zur Herstellung von Isocyanaten durch Umsetzung von Aminen mit Phosgen beansprucht, bei dem das Isocyanat als Lösungsmittel verwendet wird und die Umsetzung in einer Reaktionskolonne durchgeführt wird, wobei die kondensierte Phase am Sumpf der Reaktionskolonne ganz oder teilweise in den Verstärkungsteil der Reaktionskolonne zurückgeführt wird. Die theoretische Trennstufenzahl der Reaktionskolonne beträgt 5-60. Die Temperatur beträgt -20°C bis 300°C und der Absolutdruck 0,2-60 bar.

In US 2683160 wird ein Verfahren zur Herstellung aromatischer Isocyanate beschrieben, in dem gasförmiges Phosgen und eine Lösung eines aromatischen Amins in einem chlorierten aromatischen Kohlenwasserstoff als Lösungsmittel gleichzeitig zu einer Lösung des gewünschten Isocyanats in dem o.g. Lösungsmittel gegeben werden. Die Lösung des gewünschten Isocyanats im Lösungsmittel wird bei Siedetemperatur des Lösungsmittels gehalten, d.h. bei einer Temperatur zwischen 130°C und 300°C, wobei die Lösung unter Rückfluß kocht. Das Nebenprodukt Chlorwasserstoff und überschüssiges, unreagiertes Phosgen werden kontinuierlich über den Rückflußkühler abgezogen. Der Phosgenüberschuß beträgt mindestens 50 %, bevorzugt 80 bis 100 % der stöchiometrischen Menge. Die Aminkonzentration im Lösungsmittel beträgt 5 bis 30 %, bevorzugt 8 bis 12 %. Die Reaktion wird in einem einzigen Apparat ausgeführt, der gut gerührt und ausreichend beheizt wird.

In DE 2252068 wird ein Verfahren zur Herstellung organischer Isocyanate aus Amin und Phosgen beschrieben, wobei das auf eine Temperatur unterhalb seiner Zersetzungstemperatur bei überatmosphärischem Druck vorerhitzte Amin zuerst mit vorerhitzem Phosgen in Gegenwart eines Überschusses an einem organischen Isocyanat als Lösungsmittel bei solchen Temperaturen und Drücken zur Umsetzung gebracht wird, daß die Reaktion in einer homogenen, flüssigen Phase abläuft und anschließend in einer zweiten Stufe das intermediär gebildete organische Carbamoylchlorid bei einem niedrigeren Druck thermisch gespalten wird. In einer bevorzugten Ausführungsform wird die erste Reaktionsstufe adiabatisch ausgeführt. Die Reaktionskomponenten werden bei Temperaturen im Bereich von 120-180°C eingespeist. Die Temperatur der Reaktionsmischung am Ausgang wird bei 180-250°C und der Druck bei 100-300 atm gehalten. Die Verweilzeit der Komponenten in der ersten Reaktionszone sollte 5-150 Sekunden betragen. Die zweite Reaktionsstufe wird isotherm durchgeführt. Die Einspeisetemperatur beträgt 120-250°C, der Druck 3-30 atm. Die Verweilzeit liegt bei 3-30 Minuten. Das aus der zweiten Stufe abgezogene Isocyanat wird vor der Rückführung auf 50-80°C abgekühlt.

In US 3801518 wird ein Verfahren zur Herstellung von Toluylendiisocyanat mit einer erhöhten Acidität von 0,03-0,3 Gew.-% beschrieben. Dies erfolgt durch Phosgenierung von Toluylendiamin und anschließendes Verweilen des Reaktionsproduktes in einer Phosgenatmosphäre bei 100-200°C für einen Zeitraum von mindestens 0,08 Stunden, bevorzugt 0,08 Stunden bis 2 Stunden.

In US 3912600 wird die Verringerung der Acidität und des Gehaltes an hydrolysierbarem Chlor eines Polymethylenpolyphenylenpolyisocyanats (PMDI) durch Behandlung desselben in einem inerten, organischen Lösungsmittel bei einem Druck von 20-60 psia und einer Temperatur von 150-230°C mit der Entfernung von flüchtigen Verbindungen, sogenannten Leichtsiedern.

In GB 1196008 wird ein kontinuierliches Verfahren zur Herstellung aromatischer Mono- oder Diisocyanate durch Phosgenierung der entsprechenden Amine in einem organischen Lösungsmittel bei einer Temperatur von 120-200°C in zwei miteinander gekoppelten Reaktionsgefäßen beschrieben, wobei der Phosgenüberschuß 5-20 % im Vergleich zur berechneten stöchiometrischen Menge beträgt.

Ziel der vorliegenden Erfindung ist es, ein drei- oder mehrstufiges Verfahren zu entwickeln, welches Isocyanate mit sehr hohen chemischen Ausbeuten und hohen Raum-Zeit-Ausbeuten bei niedrigem Phosgen-Holdup liefert.

Überraschenderweise wurde gefunden, daß entgegen der allgemeinen Lehrmeinung die zweite Reaktion, die Phosgenierung des Aminhydrochlorids, bei hohen Phosgenkonzentrationen und erhöhten Temperaturen mit hoher Reaktionsgeschwindigkeit abläuft. Hohe Drücke sind daher günstig für diese Reaktion, denn hohe Drücke implizieren hohe Phosgenkonzentrationen in der Flüssigphase. Des weiteren sind erhöhte Temperaturen zur Erzielung hoher Raum-Zeit-Ausbeuten günstig. In I.I. Konstantinov, A.I. Kormucheshkina, Zhurnal Prikladnoi Khimii, 49 (3), S. 596-599, 1976) wird beschrieben, daß die Aminhydrochloridphosgenierung sehr langsam und der geschwindigkeitsbestimmende Schritt des gesamten Reaktionszyklus zum Isocyanat ist. In Konstantinov werden kinetische Messungen vorgestellt und die Reaktionsgeschwindigkeiten quantifiziert. Die Reaktionsgeschwindigkeit der Hydrochloridphosgenierung ist demnach erheblich kleiner als die des freien Amins. Wie in GB 1212249 beschrieben, führt die Bildung von Aminhydrochlorid auch zu einem Ausbeuteverlust an Isocyanat durch Harnstoffbildung. Da die Löslichkeit von Aminhydrochloriden in den entsprechenden Reaktionsgemischen und auch in den meisten kommerziell erhältlichen Lösungsmitteln sehr gering ist, wird durch Hydrochloridbildung zudem das Problem des Feststoffanfalls drastisch verschärft.

In DE 3323882 wird ein kontinuierliches Verfahren zur Heißphosgenierung von in Lösemittel suspendiertem Aminhydrochlorid oder dessen Gemischen mit Carbamoylchlorid mit überschüssigem Phosgen bei einer Temperatur zwischen 80°C und 200°C, vorzugsweise zwischen 100°C und 180°C, beschrieben. Das Verfahren ist dadurch gekennzeichnet, daß mit Hilfe einer geeigneten Trennvorrichtung die Feststoffe im Reaktor zurückgehalten werden und das während der Reaktion gebildete, im Lösemittel gelöste Isocyanat kontinuierlich aus dem Reaktor abgezogen wird. Die Abtrennung der Feststoffe wird vorzugsweise mit einem Filter bewerkstelligt. Der Nachteil dieses Verfahrens der Hydrochloridphosgenierung liegt im aufwendigem Feststoffhandling, der Verstopfungsgefahr von Rohrleitungen und insbesondere von Regelventilen und Durchflußmessern, sowie in der langen Verweilzeit, welche große Apparate mit hohem Phosgen-Holdup bedingt, sowie in den drastischen Reaktionsbedingungen und den niedrigeren Ausbeuten.

In DE 2404773 wird ein Verfahren zur Herstellung von Mono-, Di-und/oder Polyisocyanaten aus organischen primären Aminen und Phosgen beschrieben, wobei man die primären Amine mit mindestens 3 mol Phosgen pro Aminogruppe in Abwesenheit eines Lösungsmittels mischt, wobei die Reaktionsmischung gleichzeitig auf eine mittlere Teilchengröße von 1-100 µm zerkleinert wird, und die erhaltenen Suspension aus Carbaminsäurechlorid und Aminhydrochlorid in Phosgen bei Temperaturen von 100 bis 180°C, bevorzugt 120 bis 160°C und Drücken von 14 bis 55 bar, bevorzugt 21 bis 41 bar in die entsprechenden Isocyanate überführt. Bei dem Verfahren handelt es sich um ein zweistufiges Verfahren, wobei in der ersten Stufe die Ausgangsstoffe primäres Amin und Phosgen bei Temperaturen von -30°C bis 60°C, bevorzugt 0-50°C, bei Normaldruck oder vorzugsweise bei erhöhtem Druck, insbesondere bei 14 bis 55 bar, mischt und dabei gleichzeitig die Partikel auf eine mittlere Teilchengröße von 1 bis 100 µm, bevorzugt 1 bis 50 µm, zerkleinert. Das Amin wird als Flüssigkeit, Schmelze oder gegebenenfalls als Pulver dem Phosgen zugefügt. Diverse Misch- und Zerkleinerungseinrichtungen werden beschrieben. Die zweite Stufe umfaßt die Umsetzung von Aminhydrochlorid mit Phosgen zu Carbamoylchlorid und dessen Zersetzung in Isocyanat und Chlorwasserstoff in einem Druckgefäß bei Temperaturen von 100-180°C, bevorzugt 120 bis 160°C, und Drücken von 14-55 bar, bevorzugt 21 bis 41 bar. Dieses Verfahren ist technisch sehr aufwendig und nicht wirtschaftlich. In der DE-OS 156844 wird ebenfalls eine Phosgenierung einer Aminhydrochloridsuspension beschrieben, wobei diese in einer mehrstufigen Rührkesselkaskade bei erhöhter Temperatur erfolgt. Der Nachteil einer Rührkesselkaskade ist vor allem der hohe Phosgen-Holdup.

Überraschenderweise wurde nun gefunden, daß die Phosgenierung des Aminhydrochlorids bei hohen Phosgenkonzentrationen und erhöhten Temperaturen eine schnelle Reaktion ist.

Es wurde weiterhin überraschenderweise gefunden, daß bei Verwenden einer Düse als Reaktor der ersten Stufe in situ gebildetes Aminhydrochlorid und Carbamoylchlorid eine starke Übersättigung in der Reaktionsmischung aufweisen. Selbst im Falle eines Ausfallens des Aminhydrochlorids und/oder des Carbamoylchlorids weist das beanspruchte Verfahren deutliche Vorteile auf, da bei Verwendung einer Düse als Reaktor der ersten Stufe durch das Einbringen hoher Mischenergien eine sehr enge Aminhydrochlorid-Partikelgrößenverteilung mit einem sehr kleinen mittleren Partikeldurchmesser, zumeist im Nanometer- bis Mikrometerbereich, erzeugt werden kann. Günstiger ist es aber, hohe Umsätze oder möglichst Vollumsatz in der Aminhydrochloridphosgenierung zu erzielen, bevor festes Aminhydrochiorid oder Carbamoylchlorid in größeren Mengen ausfällt, da zum einen Feststoffhandling verfahrenstechnisch aufwendig ist und zu Verbackungen und Verstopfungen führen kann, und zum anderen die Phosgenierung großer und agglomerierter Aminhydrochloridpartikel, wie in der Literatur beschrieben, sehr langsam ist.

Gegenstand der Erfindung ist somit ein Verfahren zur Herstellung von Polyisocyanaten durch Umsetzung von organischen Aminen mit Phosgen in einem inerten Lösungsmittel, dadurch gekennzeichnet, daß die Umsetzung in mindestens drei Stufen erfolgt, wobei die erste Stufe in einer Düse, die zweite Stufe in mindestens einem Verweilzeitapparat und die dritte Stufe in einer oder mehreren Reaktionskolonnen durchgeführt wird und der Druck in jeder nachfolgenden Stufe geringer ist als in der vorherigen Stufe, wobei
der Druck vor der Düse der ersten Stufe 3 bis 70 bar, im Verweilzeltapparat der zweiten Stufe 2,5 bis 35 bar und in der Reaktionskolonne der dritten Stufe 2 bis 20 bar beträgt,
die Abtrennung des überschüssigen Phosgens in der Reaktionskolonne oder in einer weiteren Stufe erfolgt, und
die Temperatur im Sumpf der Reaktionskolonne 80 bis 190°C und im Kopf der Reaktionskolonne 50 bis 120°C beträgt.

In der ersten Stufe des erfindungsgemäßen Verfahrens erfolgt im wesentlichen die Umsetzung des Amins zur Carbamoylchlorid und Aminhydrochlorid, in der zweiten Stufe im wesentlichen die Umsetzung des in der ersten Stufe gebildeten Aminhydrochlorids zu Carbamoylchlorid und in der dritten Stufe im wesentlichen die Spaltung des Carbamoylchlorids zu Isocyanat und Chlorwasserstoff. Beim erfindungsgemäßen Verfahren wird die Reaktion zwischen organischem Amin und Phosgen drei- oder mehrstufig, in einem inerten Lösungsmittel, vorzugsweise Toluol oder Chlorbenzol, Dichlorbenzol oder Mischungen davon, und mit einem Phosgenüberschuß durchgeführt, wobei über jede der Stufen eine Reduzierung des Drucks erfolgt. Die erste Phosgenierstufe umfaßt eine Düse. Der Druck vor der ersten Stufe beträgt bevorzugt 80 bis 190°C, insbesondere 90 bis 150°C. Die zweite Stufe umfaßt einen oder mehrere Verweilzeitapparate, bevorzugt einen Verweilzeitapparat, der bei einem Druck von bevorzugt 15 bis 35 bar betrieben wird. Hinter der Düse wird über eine Armatur oder eine andere dafür geeignete Einrichtung auf den Druck des Verweilzeitapparats der zweiten Stufe entspannt. Es kann allerdings auch der natürliche Druckverlust der Düse zur Druckreduzierung verwendet werden.

Auch kann der Reaktor der ersten Stufe in den Reaktor der zweiten Stufe integriert werden. Insbesondere kann eine Mischdüse in die Gasphase oder bevorzugt in die Flüssigphase des zweiten Reaktors eintauchen, d.h. sich ganz oder teilweise in ihr befinden. Es kann auch der Austrag der Düse mit einer Rohrleitung, einem Tauchrohr oder einem Einsteckrohr in die Gas- oder bevorzugt in die Flüssigphase des Reaktors der zweiten Stufe geführt werden.

Die Temperatur der zweiten Stufe beträgt 80 bis 190°C, bevorzugt 90 bis 150°C. Als Reaktortyp für die zweite Stufe kommen Rohrreaktoren, Rührkessel, nicht gerührte Verweilzeitapparate, Phasentrennapparate und andere Apparate in Betracht. Der Reaktor kann auch mit einem Umpumpkreislauf versehen sein, wobei dieser wiederum einen Wärmetauscher zur Einstellung der Reaktortemperatur enthalten kann. Im Fall eines Rührkessels, eines nicht gerührten Verweilzeitapparats oder gegebenenfalls auch bei einem Phasentrennapparate wird bevorzugt die Flüssigphase standgeregelt und die Gasphase druckgeregelt in den Reaktor der dritten Stufe entspannt. Es kann jedoch auch die Gasphase, hauptsächlich enthaltend Phosgen, Chlorwasserstoff und gegebenenfalls Lösungsmittel, direkt zur Aufarbeitung, wie Auftrennung in Phosgen, Chlorwasserstoff und Lösungsmittel oder in Gemische davon, geführt werden. Der Verweilzeitreaktor der zweiten Stufe kann je nach gewünschter Verweilzeit und Kapazität der Anlage durch größere Abmessungen und Volumina gekennzeichnet sein, was unter Kosten- oder Sicherheitsaspekten, wie Phosgen-Holdup bei hohem Druck, betrachtet nachteilig sein kann. In diesem Fall kann der Reaktor der zweiten Stufe auch durch zwei oder mehrere gleichartige oder auch unterschiedliche Reaktoren und Reaktortypen, die parallel oder gegebenenfalls zur Beeinflussung des Verweilzeitspektrums auch in Serie geschaltet sein können, realisiert werden.

Der Reaktor der dritten Stufe des erfindungsgemäßen Verfahrens wird bei einem Druck von bevorzugt 3,5 bis 16 bar betrieben. Hinter dem Verweilzeitreaktor der zweiten Stufe wird über eine Armatur oder eine andere dafür geeignete Einrichtung auf den Druck des dritten Reaktors entspannt. Gegebenenfalls kann auch ein natürlicher Druckabfall genutzt werden.

In jedem Fall ist der Druck der nachfolgenden Stufe, wie oben beschrieben, so zu wählen, daß er geringer ist als der bei der vorherigen Stufe.

Die Temperatur der dritten Stufe beträgt 80 bis 190°C. Als Reaktortyp für den dritten Reaktor wird eine Reaktionskolonne verwendet, wie sie beispielsweise in WO 99/54289 beschrieben ist. Die Sumpftemperatur beträgt 80 bis 190°C und die Kopftemperatur 50 bis 120°C. Die als Reaktor der dritten Stufe eingesetzte Kolonne kann auch dazu benutzt werden, um das überschüssige Phosgen aus der Reaktionsmischung zu entfernen. Der Reaktor der dritten Stufe kann, wie der Reaktor der zweiten Stufe, ebenfalls nachteilig groß sein. In diesem Fall kann der Reaktor der dritten Stufe auch durch zwei oder mehrere gleichartige oder auch unterschiedliche Kolonnen, die in Serie geschaltet sind, realisiert werden.

Der Sumpfaustrag der Reaktionskolonne wird mit den üblichen Methoden aufgearbeitet zur Entfernung gegebenenfalls noch vorhandenen Phosgens und zur Abtrennung des Lösungsmittels. Anschließend wird im Falle der Herstellung von TDI das Roh-TDI einer Schwersiederabtrennung und Reindestillation unterworfen. Aus den Brüden der Reaktionskolonne und gegebenenfalls des Verweilzeitreaktors der zweiten Stufe werden in bekannter Weise Phosgen, Chlorwasserstoff und gegebenenfalls Lösungsmittel abgetrennt und gegebenenfalls zurückgeführt.

Als Lösungsmittel werden vorzugsweise chlorierte aromatische Kohlenwasserstoffe wie Dichlorbenzol, Chlorbenzol, Trichlorbenzol oder Mischungen davon, aromatische oder aliphatische Kohlenwasserstoffe wie Toluol, Xylol, Benzol, Pentan, Hexan, Heptan, Octan, Cyclohexan, Biphenyl, Ketonen wie 2-Butanon, Methylisobutylketon, Ester wie Diethylisophtalat, Ethylacetat, Butylacetat, Nitrile wie Acetonitril, sowie Sulfolan verwendet.

Der Vorteil des erfindungsgemäßen Verfahrens liegt insbesondere darin begründet, daß im Gegensatz zu den gemäß dem Stand der Technik üblichen Verfahren die beiden Reaktionsschritte 1) Phosgenierung des Aminhydrochlorids zu Carbamoylchlorid und 2) Zersetzung des Carbamoylchlorids zu Isocyanat und Chlorwasserstoff teilweise oder ganz in getrennten Stufen bzw. Reaktoren durchgeführt werden, wobei durch das voneinander unabhängige Einstellen des für die jeweilige Reaktion optimalen Drucks und der optimalen Temperatur sowie durch die Wahl des jeweils günstigsten Reaktordesigns sehr hohe chemische Ausbeuten, sehr hohe Raum-Zeit-Ausbeuten und gleichzeitig ein sehr niedriger Phosgen-Holdup erzielt werden können. Die Synthese kann adiabat oder isotherm ausgeführt werden. Durch das unterschiedliche Apparatedesign wird den Bedingungen der beiden Reaktionen optimal Rechnung getragen. Während die Phosgenierung des Aminhydrochlorids hohe Drücke erfordert, sind für die Zersetzung des Carbamoylchlorids niedrige Drücke vorteilhaft. Des weiteren können für den Reaktor der Phosgenierung des Aminhydrochlorids kürzere Verweilzeiten als für den der Zersetzung des Carbamoylchlorids gewählt werden, was den Gesamt-Phosgen-Holdup deutlich verringert. Weiterhin ist für die Zersetzung des Carbamoylchlorids die Entfernung des gebildeten gasförmigen Chlorwasserstoffs, insbesondere durch Strippen, vorteilhaft, weil dadurch das Carbamoylchlorid-Isocyanat-Gleichgewicht günstig auf die Seite des gewünschten Isocyanats verschoben wird. Dem kann durch die Wahl einer Reaktions kolonne als optimales Reaktordesign Rechnung getragen werden. Dabei kann auch gleichzeitig das überschüssige Phosgen entfernt werden. Dies ist in dieser Stufe nicht unbedingt erforderlich, sondern kann auch in einer weiteren Stufe durchgeführt werden. Das Entfernen von Chlorwasserstoff im Verweilzeitreaktor der zweiten Stufe wäre hingegen sehr nachteilig, da dadurch zusammen mit dem Chlorwasserstoff auch das für die Phosgenierung des Aminhydrochlorids erforderliche Phosgen entfernt würde. Verdampfendes Phosgen führte des weiteren zu einer Abkühlung der Reaktionsmischung, was zum massiven Ausfall von festem Carbamoylchlorid und Aminhydrochlorid führen könnte.

Die schnelle Reaktion zwischen Amin und Phosgen zu Carbamoylchlorid und Chlorwasserstoff sowie zu Aminhydrochlorid erfordert für gute chemische Ausbeuten durch geringe Nebenproduktbildung als Reaktionsbedingungen der ersten wie die zweite Stufe hohe Drücke, um hohe Phosgenkonzentrationen in der Flüssigphase und damit hohe Phosgenüberschüsse zu erreichen. Außerdem ist eine gute Vermischung erforderlich d.h. als Apparat soll eine Düse, eingesetzt werden. Hohe Vordrücke vor der Düse erlauben hohe Druckverluste über die Düse und damit das Einbringen großer Mischenergien.

Gelöstes Aminhydrochlorid und sehr kleine Aminhydrochloridpartikel reagieren überraschenderweise sehr schnell mit Phosgen im Verweilzeitreaktor der zweiten Stufe ab und bedürfen daher keiner langen Verweilzeit. Dabei sind hohe Phosgenkonzentrationen vorteilhaft. Höhere Drücke beeinflussen die Phosgenierung des Aminhydrochlorids nicht nachteilig, so daß vorzugsweise durch höhere Drücke hohe Phosgenkonzentrationen in der Flüssigphase eingestellt werden können. Als Apparat eignet sich besonders ein Rohreaktor, ein Rührkessel, ein nicht gerührter Verweilzeitapparat, ein Phasentrennapparat oder ein anderer Verweilzeitapparat. Vorteilhaft sind weiterhin beheizbare Ausführungen dieser Apparate um gegebenenfalls Temperaturabsenkungen durch den endothermen Zerfall des Carbamylchlorids auszugleichen. Die Reaktion der dritten Stufe, der Zerfall des Carbamoylchlorids zu Isocyanat und Chlorwasserstoff, ist eine druckabhängige Gleichgewichtsreaktion. Sie wird durch niedrige Drücke günstig auf die Seite des gewünschten Isocyanats verschoben. Da diese Reaktion kein Phosgen benötigt, stören auch die für geringe Drücke charakteristischen niedrigen Phosgenkonzentrationen in der Flüssigphase nicht, im Gegenteil, sie führen zu einem unter Sicherheitsaspekten günstigen niedrigen Phosgen-Holdup im Reaktor der dritten Stufe. Damit kann der Phosgen-Holdup insgesamt im Verfahren, aber gegebenenfalls auch in den einzelnen Apparaten, im Vergleich zu einer Rührkesselkaskade oder einem Reaktionsturm deutlich vermindert werden. Hohe Drücke hingegen sind sehr ungünstig für die Spaltung Carbamoylchlorids und erfordern lange Verweilzeiten, hohe Temperaturen und Energieverbräuche, da bei hohen Drücken das Gleichgewicht weit auf der Seite des Carbamoylchlorids liegt. Lange Verweilzeiten bedingen wiederum einen hohen Phosgen-Holdup. Als Apparat für die dritte Stufe eignet sich eine Reaktions kolonne, wie sie beispielsweise in WO 99/54289 (DE 19817691) beschrieben wird. Der Strippeffekt verschiebt das Carbamoylchlorid-Isocyanat-Gleichgewicht zusätzlich günstig auf die Seite des gewünschten Isocyanats.

Die Phosgenierung des Aminhydrochlorids muß nicht vollständig in der zweiten Stufe abgeschlossen sein, genauso kann auch schon die Zersetzung des Carbamoylchlorids in der zweiten Stufe schon einsetzen. Bevorzugt ist aber eine Auslegung des Reaktors der zweiten Stufe bezüglich Verweilzeit und anderer verfahrenstechnischer Parameter dergestalt, daß die Phosgenierung des Aminhydrochlorids möglichst weitgehend abgeschlossen und die Zersetzung des Carbamoylchlorids noch möglichst wenig fortgeschritten ist.

Werden die Phosgenierung des Aminhydrochlorids und die Zersetzung des Carbamoylchlorids gemäß dem Stand der Technik in einer Stufe oder in einem Reaktor durchgeführt, bedingt der für die Phosgenierung des Aminhydrochlorids erforderliche hohe Druck einen geringen Umsatz des Carbamoylchlorids zum Isocyanat und damit lange Verweilzeiten. Eine hohe Phosgenkonzentration und lange Verweilzeiten (große Reaktionsvolumina) bedeuten wiederum einen sehr hohen Phosgen-Holdup. Dieser liegt zugleich bei sicherheitstechnisch bedenklichen hohen Drücken und Temperaturen vor. Durch räumliche Trennung beider Reaktionen - die Phosgenierung des Aminhydrochlorids in der zweiten Phosgenierstufe bei hohem Druck und die Zersetzung des Carbamoylchlorids in der dritten Phosgenierstufe bei niedrigem Druck - werden hohe chemische Ausbeuten, hohe Raum-Zeit-Ausbeuten und vor allem ein niedriger Phosgen-Holdup im Gesamtverfahren und gegebenenfalls auch in den einzelnen Apparaten erzielt.

Eine räumliche Trennung der ersten und der zweiten Stufe ist nicht unbedingt erforderlich, da ein hoher Druck die Phosgenkonzentration in der Flüssigphase erhöht, was sowohl der ersten Reaktion zwischen Amin und Phosgen als auch der zweiten zwischen Aminhydrochlorid und Phosgen zugute kommt.

Des weiteren kann das Verfahren in allen Stufen bei erhöhter Temperatur und gegebenenfalls auch isotherm durchgeführt werde. Dadurch werden vor allem im Vergleich zur klassischen Kalt/Heiß-phosgenierung hohe Raum-Zeit-Ausbeuten und damit niedrige Phosgen-Holdups und kleinere Apparate bei gleichzeitig höheren chemischen Ausbeuten realisiert. Durch Vermeiden des Abkühlens der Reaktionsmischung in der ersten Stufe mit anschließendem Wiedererwärmen in der zweiten Stufe und den folgenden Stufen wird außerdem eine beträchtliche Menge an Energie eingespart. Durch Vermeiden des Aminhydrochloridausfalls als Feststoff können lange Verweilzeiten, wie sie zum Teil nur durch Kreislauffahrweise (Schleifenreaktoren) realisiert werden können, vermieden werden. Die Kreislauffahrweise weist zwar ebenfalls einen niedrigeren Phosgen-Holdup als beispielsweise eine Rührkesselkaskade auf, ist aber durch die verstärkte Bildung von Nebenprodukten, insbesondere Harnstoffen gekennzeichnet. Zur Vermeidung der Harnstoffbildung muß die Amin- bzw. Isocyanatkonzentration niedrig gehalten werden, was zu sehr niedrigen Raum-Zeit-Ausbeuten führt.

Die verwendeten Temperaturen und Drücke sind teilweise abhängig vom eingesetzten Amin. Ebenso sind die zu verwendenden Phosgenüberschüsse und Verweilzeiten in den einzelnen Apparaten abhängig vom eingesetzten Amin. Der Phosgenüberschuß sollte bei Diphenylmethandiisocyanaten (MDI) und/oder Polyphenylen-polymethylenpolyisocyanaten (PMDI) oder Mischungen dieser beiden mindestens 100 % des stöchiometrischen Einsatzes, bei Toluylendiisocyanat (TDI) mindestens 300 % des stöchiometrischen Einsatzes, und bei Hexamethylendiisocyanat (HDI) und Isophorondiisocyanat (IPDI) ebenfalls mindestens 300 % des stöchiometrischen Einsatzes betragen. Die Verweilzeit in der erste Stufe ist naturgemäß sehr kurz und durch die Apparateauslegung definiert. Die mittlere Verweilzeit im Verweilzeitapparat der zweiten Stufe kann zwischen einer Sekunden und 30 Minuten betragen. Bevorzugt werden mittlere Verweilzeiten von 30 Sekunden bis 10 Minuten, besonders bevorzugt sind mittlere Verweilzeiten zwischen 2 und 7 Minuten. Die mittlere Verweilzeit im Apparat der dritten Stufe (Reaktionskolonne) ist abhängig von der Zahl der Trennstufen bzw. Böden, der Wehrhöhe, dem Sumpfvolumen, der Belastung der Kolonne, und anderen verfahrenstechnischen Parametern. Sie beträgt bevorzugt maximal 1 Stunde.

Die Aminkonzentration im inerten Lösungsmittel ist ebenfalls vom eingesetzten Amin sowie vom Lösungsmittel abhängig. Bei Diphenylmethandiamin (MDA) und/oder Polyphenylen-polymethylen-polyaminen (PMDA) oder Mischungen dieser beiden kann sie 5-50 Gew.-%, bevorzugt 25-40 Gew.-%, bei Toluylendiamin (TDA) 5-50 Gew.-%, bevorzugt 15-30 Gew.-%, bei Hexamethylendiamin (HDA) 5-50 Gew.-%, bevorzugt 15-30 Gew.-% und Isophorondiisocyanat (IPDI) ebenfalls 5-50 Gew.-%, bevorzugt 15-30 Gew.-% betragen. Die Phosgenkonzentration im inerten Lösungsmittel kann 0-70 Gew.-%, bevorzugt 10-50 Gew.-% betragen. Bevorzugt wird das gleiche Lösungsmittel wie für das Amin verwendet. Es kann auch ganz auf ein Lösungsmittel verzichtet werden.

Die Erfindung soll an den nachfolgenden Beispielen näher beschrieben werden.

### Beispiele:

### 1a) Rohrreaktor als Verweilzeitapparat (I)

Eine Lösung von 0,73 kg/h Toluylendiamin (TDA) in 3,3 kg/h Chlorbenzol wurde in einer Mischdüse mit 6,2 kg/h Phosgen bei einer Temperatur von 110°C zur Reaktion gebracht. Der Druckabfall über die Düse betrug 6 bar. Die Reaktionsmischung wurde direkt in einen mit einem Doppelmantel begleitbeheizten Rohrreaktor als Verweilzeitapparat mit einer Verweilzeit von 2 Minuten bei einer Temperatur von 110°C und einem Druck von 15 bar abs. entspannt. Der Austrag des Rohrreaktors wurde über ein Regelventil in eine Glockenbodenkolonne (Reaktionskolonne) entspannt. Die Sumpftemperatur der Reaktionskolonne betrug 150°C, der Kopfdruck 3,5 bar abs. Am Kopf der Kolonne wurde ein Gemisch (5,8 kg/h) aus Phosgen (4,8 kg/h, 82 Gew.-%), Chlorwasserstoff (0,85 kg/h, 15 Gew.-%) und Chlorbenzol (0,19 kg/h, 3 Ges.-%) sowie geringe Mengen diverser Leichtsieder (CCl₄, CHCl₃, N₂, CO, CO₂) abgezogen, partiell kondensiert und zur Chlorwasserstoff/Phosgen-Trennung nach bekannter Art geführt. Ein Teil des Kondensats wurde als Rücklauf auf die Kolonne zurückgeführt. Die Kopftemperatur betrug 71°C. Am Sumpf der Kolonne wurde eine Mischung (4,4 kg/h) aus Toluylendiisocyanat (1,0 kg/h, 23 Gew.-%), Chlorbenzol (3,1 kg/h, 70 Gew.-%), Phosgen (0,27 kg/h, 6 Gew.-%), Chlorwasserstoff (0,02 kg/h, 0,5 Gew.-%) und geringe Mengen Schwersieder (0,04 kg/h, 1 Gew.-%) abgezogen. Als Sumpfumlaufverdampfer wurde ein Rohrbündelapparat mit 13 Rohren verwendet.

### 1b) Rohrreaktor als Verweilzeitapparat (II)

Eine Lösung von 0,73 kg/h Toluylendiamin (TDA) in 3,2 kg/h Chlorbenzol wurde in einer Mischdüse mit 6,2 kg/h Phosgen zur Reaktion gebracht. Der Druckabfall über die Düse betrug 8 bar. Die Reaktionsmischung wurde direkt in einen Rohrreaktor als Verweilzeitapparat mit einer Verweilzeit von 10 Sekunden bei einer Temperatur von 120°C und einem Druck von ca. 15 bar abs. entspannt. Der Austrag des Rohrreaktors strömte direkt in eine Glockenbodenkolonne. Die Sumpftemperatur der Reaktionskolonne betrug 150°C, der Kopfdruck 15 bar abs. Am Kopf der Kolonne wurde ein Gemisch (3,0 kg/h) aus Phosgen (2,1 kg/h, 71 Gew.-%) und Chlorwasserstoff (0,85 kg/h, 29 Gew.-%) sowie geringen Mengen Chlorbenzol und diverser Leichtsieder (CCl₄, CHCl₃, N₂, CO, CO₂) abgezogen, partiell kondensiert und zur Chlorwasserstoff/Phosgen-Trennung nach bekannter Art geführt. Ein Teil des Kondensats wurde als Rücklauf auf die Kolonne zurückgeführt. Am Sumpf der Kolonne wurde eine Mischung ( kg/h) aus Toluylendiisocyanat (1,0 kg/h, 14 Gew.-%), Chlorbenzol (3,2 kg/h, 45 Gew.-%), Phosgen (2,9 kg/h, 41 Gew.-%) und geringe Mengen Schwersieder (0,05 kg/h, 1 Gew.-%) abgezogen. Als Sumpfumlaufverdampfer wurde ein Rohrbündelapparat mit 13 Rohren verwendet.

### 2) Rührkessel als Verweilzeitapparat

Es wurde eine Lösung von 0,73 kg/h Toluylendiamin (TDA) in 3,3 kg/h Chlorbenzol in einer Mischdüse mit 6,2 kg/h Phosgen bei einer Temperatur von 140°C zur Reaktion gebracht. Der Druckabfall über die Düse betrug 4 bar. Die Düse war integriert in den Rohrreaktor als Verweilzeitreaktor, in den die Reaktionsmischung entspannt wurde. Der Verweilzeitreaktor war ein Rührkessel, der über den Doppelmantel auf eine Temperatur von 140°C gebracht wurde. Die Rührerdrehzahl betrug 1000 Umdrehungen pro Minute. Alternativ wurde die Düse auch außerhalb des Verweilzeitreaktors plaziert und der Austrag der Düse über ein Einsteckrohr direkt in die Flüssigphase geführt. Eine Entspannung in die Gasphase führte zu etwas geringeren Ausbeuten an Toluylendiisocyanat (TDI). Die Flüssigphase wird standgeregelt und die Gasphase druckgereglt aus dem Verweilzeitreaktor in eine Glockenbodenkolonne (Reaktionskolonne) gefahren. Der Rührkessel wurde im Druckbereich zwischen 2,5 und 35 bar abs. betrieben. Die mittlere Verweilzeit der Flüssigphase (geregelt über den Stand) betrug bis zu 30 Minuten. Der Rührkessel wurde auch mit einer externen Schleife mit Wärmetauscher (Umpumpkreislauf mit Zahnradpumpe) betrieben. Die Sumpftemperatur der Reaktionskolonne beträgt 110°C, der Kopfdruck 3,5 bar abs. Am Kopf der Kolonne wird ein Gemisch (5,0 kg/h) aus Phosgen (4,0 kg/h, 20 Gew.-%), Chlorwasserstoff (0,85 kg/h, 17 Ges.-%) und Chlorbenzol (0,16 kg/h, 3 Gew.-%) sowie geringen Mengen diverser Leichtsieder (CCl₄, CHCl₃, N₂, CO, CO₂) abgezogen, partiell kondensiert und zur Chlorwasserstoff/Phosgen-Trennung nach bekannter Art geführt. Ein Teil des Kondensats wurde als Rücklauf auf die Kolonne zurückgeführt. Die Kopftemperatur betrug 70°C. Am Sumpf der Kolonne wurde eine Mischung (5,2 kg/h) aus Toluylendiisocyanat (1,0 kg/h, 19 Gew.-%), Chlorbenzol (3,1 kg/h, 59 Gew.-%), Phosgen (1,15 kg/h, 22 Gew.-%), Chlorwasserstoff (0,02 kg/h, 0,3 Gew.-%) und geringe Mengen Schwersieder (0,02 kg/h, 0,4 Gew.-%) abgezogen. Als Sumpfumlaufverdampfer wurde ein Rohrbündelapparat mit 13 Rohren verwendet.

### 3) Phasentrennapparat

Eine Lösung von 0,74 kg/h Diaminodiphenylmethan (MDA) in 1,6 kg/h Chlorbenzol wurde in einer Mischdüse mit einer Lösung aus 1,9 kg/h Phosgen in 2,1 kg/h Chlorbenzol zur Reaktion gebracht. Der Druckabfall über die Düse betrug 5 bar. Die Düse wurde über ein Einsteckrohr in die Flüssigphase eines Phasenscheiders entspannt. Der Druck hinter der Düse betrug 12 bar, die Temperatur 115°C. Die Flüssigphase und die Gasphase wurden getrennt in eine Glockenbodenkolonne (Reaktionskolonne) gefahren. Die mittlere Verweilzeit der Flüssigphase betrug ca. 3 Minuten. Die Sumpftemperatur der Reaktionskolonne betrug 115°C, der Kopfdruck 5 bar abs. Am Kopf der Kolonne wurde ein Gemisch (1,5 kg/h) aus Phosgen (0,73 kg/h, 50 Gew.-%), Chlorwasserstoff (0,50 kg/h, 33 Gew.-%) und Chlorbenzol (0,24 kg/h, 16 Gew.-%) sowie geringen Mengen diverser Leichtsieder (CCl₄, CHCl₃, N₂, CO, CO₂) abgezogen, partiell kondensiert und zur Chlorwasserstoff/Phosgen-Trennung nach bekannter Art geführt. Ein Teil des Kondensats wurde als Rücklauf auf die Kolonne zurückgeführt. Die Kopftemperatur betrug 110°C. Am Sumpf der Kolonne wurde eine Mischung (4,9 kg/h) aus Methylendiphenyldiisocyanat (MDI, 0,93 kg/h, 19 Gew.-%), Chlorbenzol (3,5 kg/h, 71 Gew.-%), Phosgen (0,43 kg/h, 9 Gew.-%) und Chlorwasserstoff (0,05 kg/h, 1,0 Gew.-%) abgezogen. Als Sumpfumlaufverdampfer wurde ein Rohrbündelapparat mit 13 Rohren verwendet.

## Patentansprüche

1. Verfahren zur Herstellung von Polyisocyanaten durch Umsetzung von organischen Aminen mit Phosgen in einem inerten Lösungsmittel, **dadurch gekennzeichnet, daß** die Umsetzung in mindestens drei Stufen erfolgt, wobei die erste Stufe in in einer Düse, die zweite Stufe in mindestens einem Verweilzeitapparat und die dritte Stufe in einer oder mehreren Reaktionskolonnen durchgeführt wird und der Druck in jeder nachfolgenden Stufe geringer ist als in der vorherigen Stufe, wobei
- der Druck vor der Düse der ersten Stufe 3 bis 70 bar, im Verweilzeitapparat der zweiten Stufe 2,5 bis 35 bar und in der Reaktionskolonne der dritten Stufe 2 bis 20 bar beträgt,
- die Abtrennung des überschüssigen Phosgens in der Reaktionskolonne oder in einer weiteren Stufe erfolgt, und
- die Temperatur im Sumpf der Reaktionskolonne 80 bis 190°C und im Kopf der Reaktionskolonne 50 bis 120°C beträgt.

2. Verfahren nach Anspruch 1, **dadurch gekennzeichnet, dass** die Polyisocyanate Diphenylmethandiisocyanat (MDI), Polyphenylen-polymethylen-polyisocyanat (PMDI) oder Mischungen dieser beiden, Toluylendiisocyanat (TDI), Hexamethylendiisocyanat (HDI) oder um Isophorondiisocyanat (IPDI) sind.

3. Verfahren nach Anspruch 1, **dadurch gekennzeichnet, dass** als Apparat für die zweite Stufe ein Rohrreaktor, ein Rührkessel, ein nicht gerührter Verweilzeitapparat oder ein Phasentrennapparat für Gas- und Flüssigphase verwendet wird.

4. Verfahren nach den Ansprüchen 1-3, **dadurch gekennzeichnet, dass** die Verweilzeit im Verweilzeitapparat der zweiten Stufe 1 Sekunde bis 30 Minuten, bevorzugt 30 Sekunden bis 10 Minuten, besonders bevorzugt 2 bis 7 Minuten beträgt.

5. Verfahren nach den Ansprüchen 1-4 **dadurch gekennzeichnet, dass** der Verweilzeitreaktor der zweiten Stufe durch zwei oder mehrere gleichartige oder auch unterschiedliche Reaktoren, die parallel oder in Serie geschaltet sind, realisiert wird.

6. Verfahren nach Anspruch 1, **dadurch gekennzeichnet, dass** die Phosgenabtrennung im Apparat der dritten Stufe, der Reaktionskolonne, durchgeführt wird.

7. Verfahren nach Anspruch 1, **dadurch gekennzeichnet, dass** vom Druck des Reaktors der ersten Stufe über ein Regelventil oder eine andere durch einen Druckverlust gekennzeichnete Vorrichtung auf den Druck des Reaktors der zweiten Stufe reduziert wird.

8. Verfahren nach Anspruch 1, **dadurch gekennzeichnet, dass** vom Druck des Reaktors der zweiten Stufe über ein Regelventil oder eine andere durch einen Druckverlust gekennzeichnete Vorrichtung auf den Druck des Reaktors der dritten Stufe reduziert wird.

9. Verfahren nach Anspruch 1, **dadurch gekennzeichnet, dass** der Reaktor der ersten Stufe in den Reaktor der zweiten Stufe integriert ist.

10. Verfahren nach den Ansprüchen 1 bis 8, **dadurch gekennzeichnet, dass** der Druck vor der Düse der ersten Stufe 15-45 bar, der Druck im Verweilzeitreaktor der zweiten Stufe 15-35 bar, und der Druck in der Reaktionskolonne der dritten Stufe 3,5-16 bar beträgt.

11. Verfahren nach den Ansprüchen 1 bis 9, **dadurch gekennzeichnet, dass** die Temperatur in der ersten, zweiten und dritten Stufe jeweils 80-190°C, bevorzugt 90-150°C beträgt.

12. Verfahren nach den Ansprüchen 1 bis 10, **dadurch gekennzeichnet, dass** als inertes Lösungsmittel ein aromatischer Kohlenwasserstoff wie Toluol oder bevorzugt ein chlorierter aromatischer Kohlenwasserstoff wie Chlorbenzol, ortho-Dichlorbenzol oder Trichlorbenzol oder Mischungen davon verwendet wird.

## Claims

1. A process for preparing polyisocyanates by reacting organic amines with phosgene in an inert solvent, wherein the reaction is carried out in at least three stages, with the first stage being carried out in a nozzle, the second stage in at least one residence apparatus and the third stage in one or more reaction columns and the pressure in each successive stage being lower than that in the previous stage, where
- the pressure upstream of the nozzle of the first stage is from 3 to 70 bar, that in the residence apparatus of the second stage is from 2.5 to 35 bar and that in the reaction column of the third stage is from 2 to 20 bar,
- the excess phosgene is separated off in the reaction column or in a further stage and
- the temperature at the bottom of the reaction column is from 80 to 190°C and that at the top of the reaction column is from 50 to 120°C.

2. The process according to claim 1, wherein the polyisocyanate is diphenylmethane diisocyanate (MDI), polyphenylene-polymethylene polyisocyanate (PMDI) or a mixture of these two, tolylene diisocyanate (TDI), hexamethylene diisocyanate (HDI) or isophorone diisocyanate (IPDI).

3. The process according to claim 1, wherein a tube reactor, a stirred vessel, an unstirred residence apparatus or a phase separation apparatus for gas and liquid phases is used as apparatus for the second stage.

4. The process according to any of claims 1-3, wherein the residence time in the residence apparatus of the second stage is from 1 second to 30 minutes, preferably from 30 seconds to 10 minutes, particularly preferably from 2 to 7 minutes.

5. The process according to any of claims 1-4, wherein the residence reactor of the second stage is configured as two or more reactors of the same or different types which are connected in parallel or in series.

6. The process according to claim 1, wherein the phosgene is separated off in the apparatus of the third stage, the reaction column.

7. The process according to claim 1, wherein the pressure is reduced from the pressure of the reactor of the first stage to the pressure of the reactor of the second stage by means of a regulating valve or some other device **characterized by** a pressure drop.

8. The process according to claim 1, wherein the pressure is reduced from the pressure of the reactor of the second stage to the pressure of the reactor of the third stage by means of a regulating valve or some other device **characterized by** a pressure drop.

9. The process according to claim 1, wherein the reactor of the first stage is integrated into the reactor of the second stage.

10. The process according to any of claims 1 to 8, wherein the pressure upstream of the nozzle of the first stage is 15-45 bar, the pressure in the residence reactor of the second stage is 15-35 bar, and the pressure in the reaction column of the third stage is 3.5-16 bar.

11. The process according to any of claims 1 to 9, wherein the temperature in the first, second and third stages is in each case 80-190°C, preferably 90-150°C.

12. The process according to any of claims 1 to 10, wherein an aromatic hydrocarbon such as toluene or preferably a chlorinated aromatic hydrocarbon such as chlorobenzene, ortho-dichlorobenzene or trichlorobenzene or a mixture thereof is used as inert solvent.

## Revendications

1. Procédé de fabrication de polyisocyanates par réaction d'amines organiques avec du phosgène dans un solvant inerte, **caractérisé en ce que** la réaction a lieu en au moins trois étapes, la première étape étant réalisée dans une buse, la deuxième étape dans au moins un appareil à temps de séjour et la troisième étape dans une ou plusieurs colonnes de réaction, et la pression dans chaque étape ultérieure étant inférieure à celle de l'étape précédente,
- la pression avant la buse de la première étape étant de 3 à 70 bar, dans l'appareil à temps de séjour de la deuxième étape de 2,5 à 35 bar et dans la colonne de réaction de la troisième étape de 2 à 20 bar,
- la séparation du phosgène en excès ayant lieu dans la colonne de réaction ou à une étape ultérieure et
- la température au fond de la colonne de réaction étant de 80 à 190°C et à la tête de la colonne de réaction de 50 à 120°C.

2. Procédé selon la revendication 1, **caractérisé en ce que** les polyisocyanates sont le diisocyanate de diphénylméthane (MDI), le polyisocyanate de polyphénylène-polyméthylène (PMDI) ou des mélanges de ces deux composés, le diisocyanate de toluylène (TDI), le diisocyanate d'hexaméthylène (HDI) ou le diisocyanate d'isophorone (IPDI).

3. Procédé selon la revendication 1, **caractérisé en ce qu'**un réacteur tubulaire, une cuve agitée, un appareil à temps de séjour non agité ou un appareil de séparation de phases pour phase vapeur et phase liquide est utilisé en tant qu'appareil pour la deuxième étape.

4. Procédé selon les revendications 1 à 3, **caractérisé en ce que** temps de séjour dans l'appareil à temps de séjour de la deuxième étape est de 1 seconde à 30 minutes, de préférence de 30 secondes à 10 minutes, de manière particulièrement préférée de 2 à 7 minutes.

5. Procédé selon les revendications 1 à 4, **caractérisé en ce que** le réacteur à temps de séjour de la deuxième étape est réalisé par deux réacteurs ou plus identiques ou différents, qui sont connectés en parallèle ou en série.

6. Procédé selon la revendication 1, **caractérisé en ce que** la séparation du phosgène est réalisée dans l'appareil de la troisième étape, la colonne de réaction.

7. Procédé selon la revendication 1, **caractérisé en ce que** la pression du réacteur de la première étape est réduite à la pression du réacteur de la deuxième étape par une vanne de régulation ou un autre dispositif **caractérisé par** une perte de charge.

8. Procédé selon la revendication 1, **caractérisé en ce que** la pression du réacteur de la deuxième étape est réduite à la pression du réacteur de la troisième étape par une vanne de régulation ou un autre dispositif **caractérisé par** une perte de charge.

9. Procédé selon la revendication 1, **caractérisé en ce que** le réacteur de la première étape est intégré dans le réacteur de la deuxième étape.

10. Procédé selon les revendications 1 à 8, **caractérisé en ce que** la pression avant la buse de la première étape est de 15 à 45 bar, la pression dans le réacteur à temps de séjour de la deuxième étape est de 15 à 35 bar, et la pression dans la colonne de réaction de la troisième étape est de 3,5 à 16 bar.

11. Procédé selon les revendications 1 à 9, **caractérisé en ce que** la température dans la première, deuxième et troisième étape est à chaque fois de 80 à 190°C, de préférence de 90 à 150°C.

12. Procédé selon les revendications 1 à 10, **caractérisé en ce qu'**un hydrocarbure aromatique, tel que le toluène, ou de préférence un hydrocarbure aromatique chloré, tel que le chlorobenzène, l'ortho-dichlorobenzène ou le trichlorobenzène ou leurs mélanges, est utilisé en tant que solvant inerte.
